# EUROPEAN PATENT APPLICATION

(11) **EP 4 316 495 A1**
(43) Date of publication of application: **07.02.2024**
(21) Application number: 22774339.0
(22) Date of filing: 25.03.2022
(51) Int. Cl.: A61K 31/7088, A61P 19/02

(54) **NUCLEIC ACID MOLECULE CONJUGATE HAVING PROLONGED IN VIVO HALF-LIFE**

(30) Priority: 25.03.2021 CN 202110319264
(71) Applicant: Aptacure Therapeutics Limited, Hong Kong (HK)
(72) Inventor: ZHANG, Ge, Hong Kong (CN); HE, Yixin, Hong Kong Science Park, No. 15 Science Park West Avenue, Pak Shek Kok, New Territories Hong Kong (CN)
(74) Representative: Nederlandsch Octrooibureau
(86) International application number: PCT/CN2022/082996
(87) International publication number: WO 2022/199685

(57) **Abstract**

The invention relates to the field of biomedicine. In particular, the present invention relates to a nucleic acid molecule conjugate with extended *in vivo* half-life, wherein the nucleic acid molecule is conjugated to a fatty acid and a coumarin derivative. More specifically, the nucleic acid molecule is an aptamer, in particular an aptamer against sclerostin.

## Description

### Technical field

The invention relates to the field of biomedicine. In particular, the present invention relates to a nucleic acid molecule conjugate with extended *in vivo* half-life, wherein the nucleic acid molecule is conjugated to a fatty acid and a coumarin derivative. More specifically, the nucleic acid molecule is an aptamer, in particular an aptamer against sclerostin.

### Background of the invention

Aptamers are short single-stranded oligonucleotides which bind to their targets through conformational complementarity (Ellington and Szostak 1990, Tuerk and Gold 1990). Aptamers can be tailored selected against positive and negative targets. Compared to therapeutic antibodies, aptamers possess similar affinity and specificity, but have some important advantages. For immunogenicity, aptamers are not recognized by the immune system as foreign and do not stimulate a negative immune response because of the small size (Keefe, Pai et al. 2010). As for production and cost, aptamers are identified in vitro under various selection conditions and can be easily synthesized by chemical methods, so production is less expensive and less risky (Banerjee 2010). As for stability, aptamers have an indefinite shelf life as they are temperature resistant and can tolerate transportation without any special requirements for cooling, eliminating the need for a continuous cold chain (Jayasena 1999). Pegaptanib, an aptamer against vascular endothelial growth factor (VEGF) for the treatment of age-related macular degeneration has been successfully used in clinic (Jellinek, Green et al. 1994, Ruckman, Green et al. 1998, Ng and Adamis 2006, Que-Gewirth and Sullenger 2007).

However, unmodified aptamers are susceptible to degradation by enzymes widely present in the body. More importantly, because the molecular weight of unmodified aptamers is not large enough, they will be easily filtered and excreted by the kidney. This makes the half-life of unmodified aptamers very short and has limited therapeutic effect when used clinically. Clinically, nucleic acid aptamers are often covalently modified with PEG having large molecular weight (20kDa, 40kDa) to allow their overall molecular weight greater than the glomerular filtration molecular weight cutoff (30-50kDa) to increase the circulating half-life of the molecules in the body. However, the large molecular weight of the PEG component makes the proportion of aptamers in the active ingredients very small. At the same time, due to solubility and solution fluidity problems, the presence of PEG limits the maximum dosage and limits clinical application of many aptamer drugs.

Therefore, extending the circulation half-life of aptamers through chemical modification with small molecules while improving the enzyme stability of aptamers themselves, has become an important research direction in the development of aptamer drugs. Therefore, there remains a need in the art for modified aptamers with extended in vivo half-life and enhanced in vivo efficacy.

Osteoporosis is a disease with reduced bone mass and bone strength, leading to an increased risk of bone fractures (Hamersma, Gardner et al. 2003). A variety of drugs for osteoporosis treatment are mainly anti-resorptive agents, which inhibit bone resorption to prevent further bone loss (Russell, Watts et al. 2008, Pennypacker, Duong et al. 2011). Parathyroid hormone (PTH) peptide is the only available anabolic agent for stimulating bone formation to reverse established osteoporosis (Compston 2007, Greenspan, Bone et al. 2007). Unfortunately, long term treatment using PTH leads to risk of osteosarcoma (Whitfield 2001, Orwoll, Scheele et al. 2003). Therefore, alternative anabolic drugs which can promote bone formation without any adverse effects are seriously needed.

Sclerostin is a promising target for developing therapeutics in established osteoporosis (Rey and Ellies 2010). Humanized monoclonal antibody against human sclerostin has been reported to promote bone formation and increase bone mass with good tolerance in clinical trials. However, there are several major concerns for therapeutic antibodies, including high immunogenicity (Padhi, Jang et al. 2011, Padhi, Allison et al. 2014), expensive and laborious to produce (Baker 2015, Bradbury and Pluckthun 2015, Groff, Brown et al. 2015), unstable which requires a continuous cold chain for transportation and storage (Jayasena 1999). Therefore, an alternative anti-sclerostin agent with no immunogenicity, easy production, low cost and high stability is desirable for bone anabolic therapy.

### Summary of the invention

The present invention at least provides the following embodiments:
Embodiment 1. A nucleic acid molecule conjugate with extended in vivo half-life, wherein the nucleic acid molecule is conjugated to a fatty acid and/or a coumarin derivative.
Embodiment 2. The nucleic acid molecule conjugate of Embodiment 1, wherein the fatty acid is selected from dodecanedioic acid, palmitic acid (PA), tetradecanedioic acid, hexadecanedioic acid, stearic acid (SA), octadecanedioic acid, lauric acid, acid, eicosapentaenoic acid (EPA), docosahexaenoic acid (DHA), and arachidonic acid (ARA), preferably, the fatty acid is dodecanedioic acid.
Embodiment 3. The nucleic acid molecule conjugate of Embodiment 1 or 2, wherein the coumarin derivative is selected from 4-hydroxycoumarin, 3-acetyl-6-carboxycoumarin, warfarin, (2-oxo-2H-chromen-3-yl)acetic acid, [(8-acetyl-4-methyl- 2-oxo-2H-chromen-7-yl)oxy] acetic acid, coumarin-3-carboxylic acid, N-(4-methyl-7-coumarin) oxalamide, 7-(carboxymethyl )-4-methylcoumarin, 7-methoxycoumarin-3-carboxylic acid, and 6-methoxy-2-oxo-2H-chromene-3-carboxylic acid, preferably, the coumarin derivative is 4-hydroxycoumarin.
Embodiment 3. The nucleic acid molecule conjugate of any one of Embodiments 1-3, wherein the fatty acid, such as dodecanedioic acid, is conjugated to the 5' end of the nucleic acid molecule; or the coumarin derivative, such as 4-hydroxycoumarin, is conjugated to the 5' end of the nucleic acid molecule; or the fatty acid, such as dodecanedioic acid, and the coumarin derivative, such as 4-hydroxycoumarin, are conjugated to the 5' end of the nucleic acid molecule.
Embodiment 5. The nucleic acid molecule conjugate of any one of Embodiments 1-4, wherein the fatty acid, such as dodecanedioic acid, is conjugated to the nucleic acid molecule through a linker; or the coumarin derivative, such as 4-hydroxycoumarin, is conjugated to the nucleic acid molecule via a linker; or the fatty acid, such as dodecanedioic acid, and the coumarin derivative, such as 4-hydroxycoumarin, are conjugated to the nucleic acid molecule through a linker.
Embodiment 6. The nucleic acid molecule conjugate of any one of Embodiments 1-5, wherein the nucleic acid molecule is a DNA molecule, an RNA molecule, or a DNA/RNA hybrid molecule.
Embodiment 7. The nucleic acid molecule conjugate of any one of Embodiments 1-6, wherein the nucleic acid molecule is double-stranded; or the nucleic acid molecule is single-stranded; or the nucleic acid molecule contains a single-stranded portion and a double-stranded portion.
Embodiment 8. The nucleic acid molecule conjugate of any one of Embodiments 1-7, wherein the nucleic acid molecule is about 1 to about 250 bp/nt, about 1 to about 200 bp/nt, about 1 to about 150 bp/nt, about 1 to about 100 bp/nt, about 1 to about 50 bp /nt, about 1 to about 30bp/nt, about 1 to about 20bp/nt, about 1 to about 15bp/nt, about 1 to about 10bp/nt in length.
Embodiment 9. The nucleic acid molecule conjugate of any one of Embodiments 1-8, wherein the nucleic acid molecule is an aptamer, siRNA, antisense RNA, or shRNA, preferably, the nucleic acid molecule is an aptamer.
Embodiment 10. The nucleic acid molecule conjugate of any one of Embodiments 1-9, wherein the nucleic acid molecule comprises one or more modifications that confers enhanced nuclease resistance to the nucleic acid molecule.
Embodiment 11. The nucleic acid molecule conjugate of Embodiment 10, wherein the modification includes a 3' inverted deoxythymidine (3' idT) modification.
Embodiment 12. The nucleic acid molecule conjugate of Embodiment 10, wherein the modification includes substituting one or more naturally occurring nucleotides with modified nucleotides selected from the group consisting of 2'-fluoro, 2'-methoxyethyl, 2'-methoxy or 2' allyloxy modified nucleotides, preferably 2'-methoxy modified nucleotides.
Embodiment 13. The nucleic acid molecule conjugate of Embodiment 10, wherein the modification includes an internucleotide modification, such as an internucleotide phosphorothioate bond modification.
Embodiment 14. The nucleic acid molecule conjugate of Embodiment 10, wherein the aptamer comprises a 2'-methoxy (2'-OMe) modification, and/or a 3' inverse deoxythymidine (3' idT) modification.
Embodiment 15. The nucleic acid molecule conjugate of any one of Embodiments 1-14, wherein the in vivo half-life of the nucleic acid molecule conjugate is at least 2 times, at least 5 times, at least 10 times, at least 25 times, at least 50 times, at least 100 times, at least 200 times or more as compared to a corresponding nucleic acid molecule that does not comprise the conjugated fatty acid and/or coumarin derivative
Embodiment 16. A aptamer conjugate against sclerostin, which comprises an aptamer against sclerostin conjugated to a fatty acid and/or a coumarin derivative,
   wherein the aptamer comprises
      i) a nucleotide sequence having at least about 90% identity, at least about 91% identity, at least about 90% identity, about 92% identity, at least about 93% identity, at least about 94% identity, or at least about 95% identity to any one of SEQ ID NOs:1-17, or
      ii) at least 30, at least 35, at least 40, at least 45, at least 50, or more consecutive nucleotides within any one of SEQ ID NOs: 1-17,
   wherein the aptamer specifically binds to sclerostin.
Embodiment 17. The aptamer conjugate of Embodiment 16, wherein the aptamer comprises a nucleotide sequence of any one of SEQ ID NOs: 1-17 and 19-25.
Embodiment 18. The aptamer conjugate of Embodiment 16 or 17, wherein the aptamer is conjugated to a fatty acid and a coumarin derivative.
Embodiment 19. The aptamer conjugate of any one of Embodiments 16-18, wherein the fatty acid is selected from dodecanedioic acid, palmitic acid (PA), tetradecanedioic acid, hexadecanedioic acid, stearic acid (SA), octadecanedioic acid, lauric acid, acid, eicosapentaenoic acid (EPA), docosahexaenoic acid (DHA), and arachidonic acid (ARA), preferably, the fatty acid is dodecanedioic acid.
Embodiment 20. The aptamer conjugate of any one of Embodiments 16-19, wherein the coumarin derivative is selected from 4-hydroxycoumarin, 3-acetyl-6-carboxycoumarin, warfarin, (2-oxo-2H-chromen-3-yl)acetic acid, [(8-acetyl-4-methyl- 2-oxo-2H-chromen-7-yl)oxy] acetic acid, coumarin-3-carboxylic acid, N-(4-methyl-7-coumarin) oxalamide, 7-(carboxymethyl )-4-methylcoumarin, 7-methoxycoumarin-3-carboxylic acid, and 6-methoxy-2-oxo-2H-chromene-3-carboxylic acid, preferably, the coumarin derivative is 4-hydroxycoumarin.
Embodiment 21. The aptamer conjugate of any one of Embodiments 16-20, wherein the fatty acid, such as dodecanedioic acid, is conjugated to the 5' end of the aptamer; or the coumarin derivative, such as 4-hydroxycoumarin, is conjugated to the 5' end of the aptamer; or the fatty acid, such as dodecanedioic acid, and the coumarin derivative, such as 4-hydroxycoumarin, are conjugated to the 5' end of the aptamer.
Embodiment 22. The aptamer conjugate of any one of Embodiments 16-21, wherein the fatty acid, such as dodecanedioic acid, is conjugated to the aptamer through a linker; or the coumarin derivative, such as 4-hydroxycoumarin, is conjugated to the aptamer via a linker; or the fatty acid, such as dodecanedioic acid, and the coumarin derivative, such as 4-hydroxycoumarin, are conjugated to the aptamer through a linker.
Embodiment 23. The aptamer conjugate of any one of Embodiments 16-22, wherein the aptamer has a Kd to sclerostin of less than 100 nM, preferably less than 50 nM, preferably less than 40 nM, preferably less than 30 nM, preferably less than 20 nM, preferably less than 10 nM or less.
Embodiment 24. The aptamer conjugate of any one of Embodiments 16-23, wherein the aptamer can inhibit the biological activity of sclerostin.
Embodiment 25. The aptamer conjugate of any one of Embodiments 16-24, wherein the aptamer can block the antagonistic effect of sclerostin in a cell-based Wnt signaling assay.
Embodiment 26. The aptamer conjugate of any one of Embodiments 16-25, wherein the aptamer inhibits the biological activity of sclerostin, such as inhibits the antagonistic effect of sclerostin on the Wnt signaling pathway with a EC50 value of less than 100 µg/ml, preferably less than 50 µg/ml, preferably less than 40 µg/ml, preferably less than 30 µg/ml, preferably less than 20 µg/ml, preferably less than 10 µg/ml or less.
Embodiment 27. The aptamer conjugate of any one of Embodiments 16-26, wherein the aptamer comprises one or more modifications that confers enhanced nuclease resistance to the nucleic acid molecule.
Embodiment 28. The aptamer conjugate of Embodiment 27, wherein the modification includes a 3' inverted deoxythymidine (3' idT) modification.
Embodiment 29. The aptamer conjugate of Embodiment 27, wherein the modification includes substituting one or more naturally occurring nucleotides with modified nucleotides selected from the group consisting of 2'-fluoro, 2'-methoxyethyl, 2'-methoxy or 2' allyloxy modified nucleotides, preferably 2'-methoxy modified nucleotides.
Embodiment 30. The aptamer conjugate of Embodiment 27, wherein the modification includes an internucleotide modification, such as an internucleotide phosphorothioate bond modification.
Embodiment 31. The aptamer conjugate of Embodiment 27, wherein the aptamer comprises a 2'-methoxy (2'-OMe) modification, and/or a 3' inverse deoxythymidine (3' idT) modification.
Embodiment 32. A method for treating sclerostin-related diseases, the method comprises administering a therapeutically effective amount of the aptamer conjugate against sclerostin of any one of Embodiments 16-31 to a subject in need thereof, for example, the subject is a human.
Embodiment 33. The method of Embodiment 32, wherein the sclerostin-related disease is selected from osteoporosis, osteopenia, osteomalacia, osteogenesis imperfecta (OI), ischemic osteonecrosis, rheumatoid arthritis, fracture, osteoarthritis and myeloma, hypophosphatemic rickets and triple negative breast cancer.
Embodiment 34. A pharmaceutical composition comprising at least one aptamer conjugate against sclerostin of any one of Embodiments 16-31, and a pharmaceutically acceptable carrier or excipient.
Embodiment 35. Use of the aptamer conjugate against sclerostin of any one of Embodiments 16-31 or the pharmaceutical composition of Embodiment 34 in the preparation of a medicine for treating sclerostin-related diseases.
Embodiment 36. The use of Embodiment 35, wherein the sclerostin-related disease is selected from osteoporosis, osteopenia, osteomalacia, osteogenesis imperfecta (OI), ischemic osteonecrosis, rheumatoid arthritis, fracture, osteoarthritis and myeloma, hypophosphatemic rickets and triple negative breast cancer.

### Brief description of the drawings

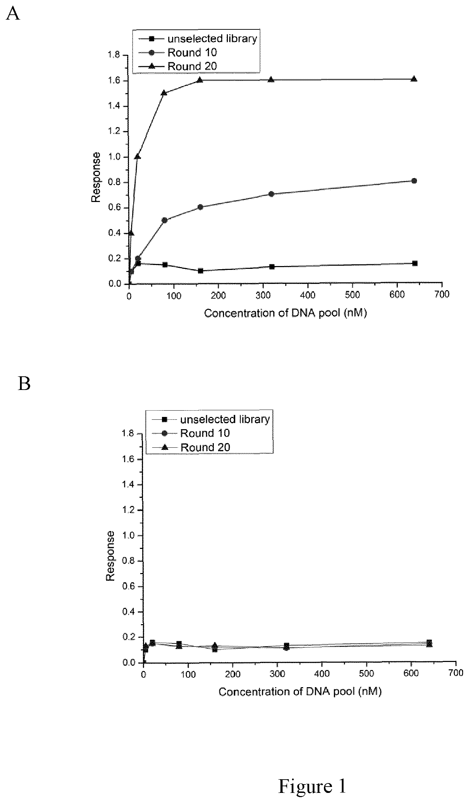

Figure 1. The enrichment of high affinity aptamers against sclerostin through SELEX. (A) The binding affinity of the enriched ssDNA and unselected library to sclerostin. (B) The binding affinity of the enriched ssDNA library and unselected library to control proteins.

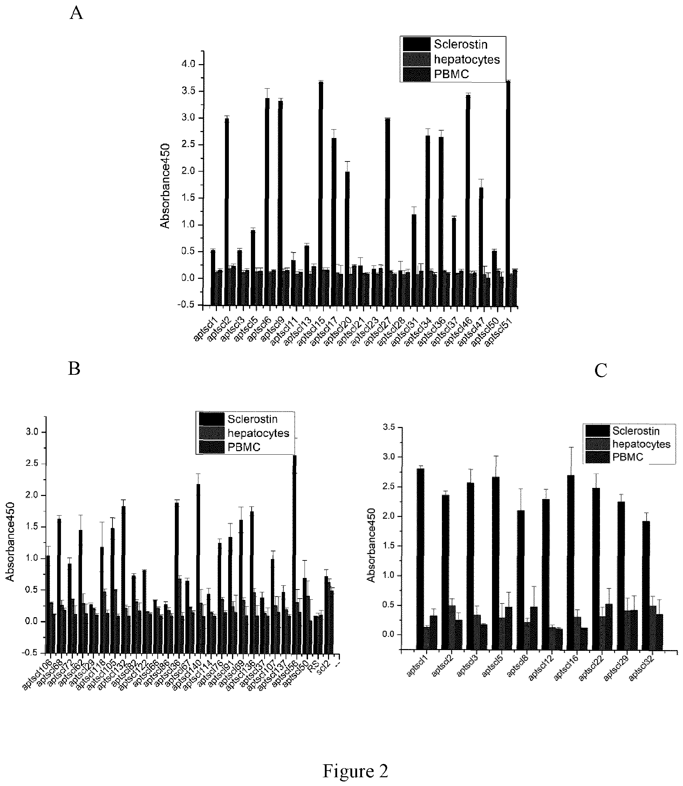

Figure 2. The specificity characterization of the aptamer candidates. Compared with binding to hepatocytes and PBMC, the aptamer candidates show high selectivity for human sclerostin.

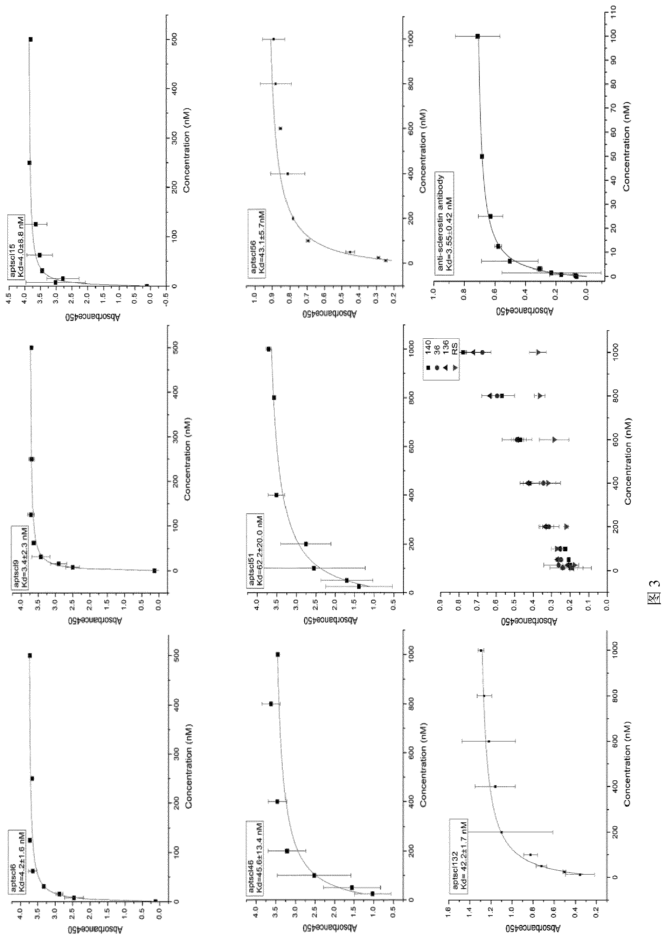

Figure 3. Binding affinity of the aptamer candidates identified from ssDNA library with a 40 nt random region to recombinant human sclerostin. The dissociation constant values (Kd) for aptamer candidates and antibody to sclerostin was calculated by non-linear curve fitting analysis. The Kd value for each aptamer candidate to sclerostin was: 4.2 nM for aptscl 6, 3.4 nM for aptscl 9, 45.6 nM for aptscl 15, 43.1 for aptscl 46, 43.1 nM for aptscl 56 and 42.2 nM for aptscl 132, respectively. The Kd value was 3.55 nM for anti-sclerostin antibody to sclersotin.

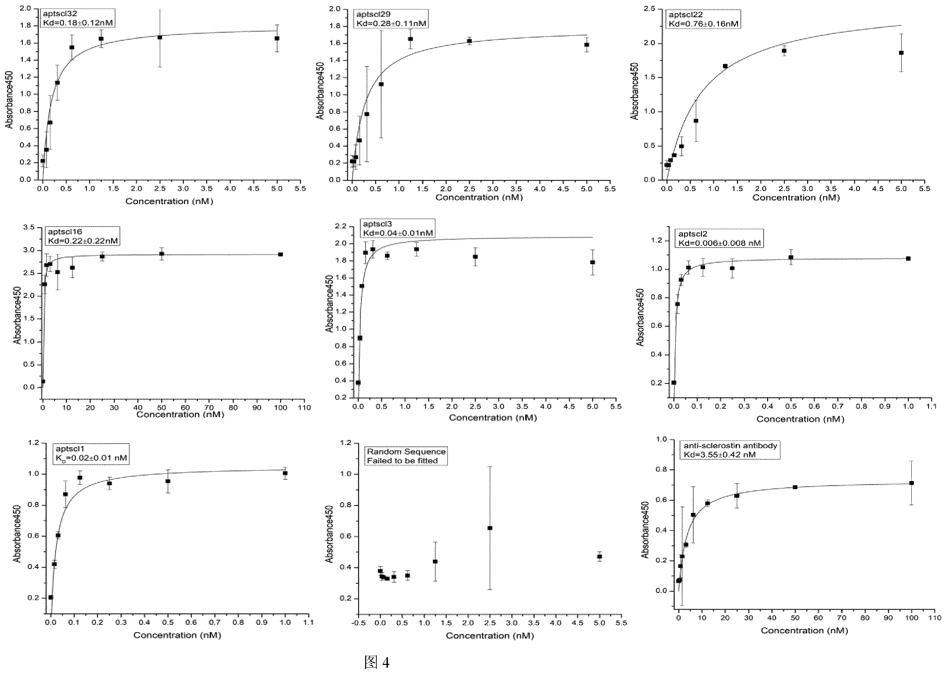

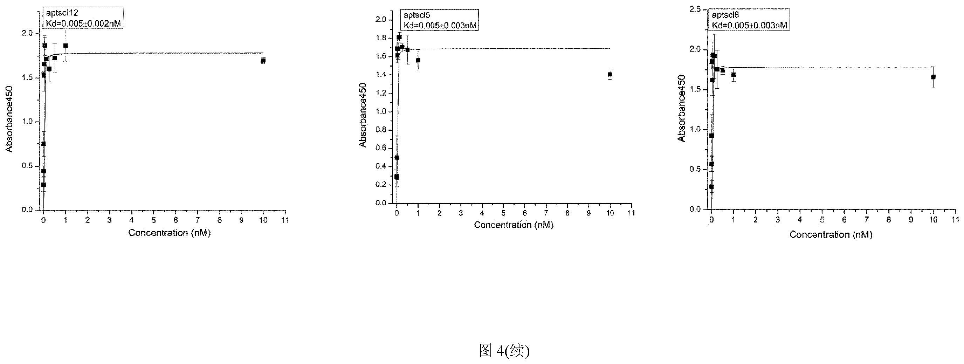

Figure 4. Binding affinity of the aptamer candidates identified from ssDNA library with a 25 nt random region to recombinant human sclerostin. The dissociation constant value (Kd) for each candidate was calculated by non-linear curve fitting analysis. The Kd value for each aptamer candidate and antibody was: 0.18 nM for aptscl 32, 0.28 nM for aptscl 29, 0.76 nM for aptscl 22, 0.22 for aptscl 16, 0.04 nM for aptscl 3, 0.006 nM for aptscl 2 and 0.02 nM for aptscl 1, respectively. The Kd value was 3.55 nM for anti-sclerostin antibody to sclersotin.

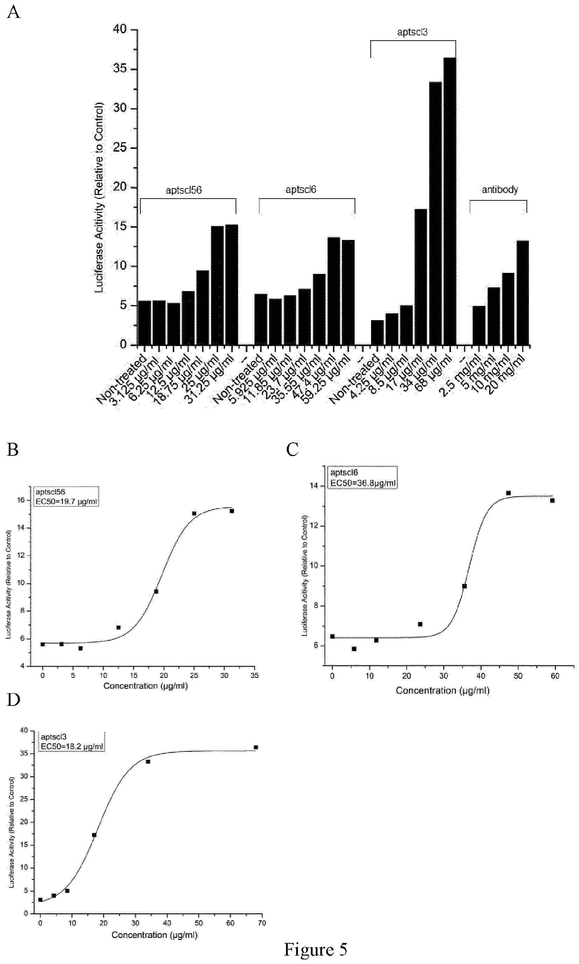

Figure 5. Inhibition potency assessment of the aptamer candidates and antibody using TOP-Wnt-induced luciferase reporter assay. (A) The luciferase activity of Wnt signaling in MC3T3-E1 cells treated with aptamer candidates in comparison with antibody. Aptscl 56, aptscl 6, aptscl 3 and anti-sclerostin antibody can effectively inhibit sclerostin's antagonistic effect on Wnt signaling and release the Wnt-induced luciferase activity. The response was stable when the concentrations of aptscl 56 and 6 reached to 25 and 47.4 µg/ml, respectively. When treated with antibody, the response was still not stable with the concentration increasing to 20 mg/ml in this experiment. (B) The inhibition potency analysis of aptscl 56. The EC50 for aptscl56 was 19.7 µg/ml. (C) The inhibition potency analysis of aptscl 6. The EC50 for aptscl 6 was 36.8 µg/ml. (D) The inhibition potency analysis of aptscl 3. The EC50 for aptscl 3 was 18.2 µg/ml.

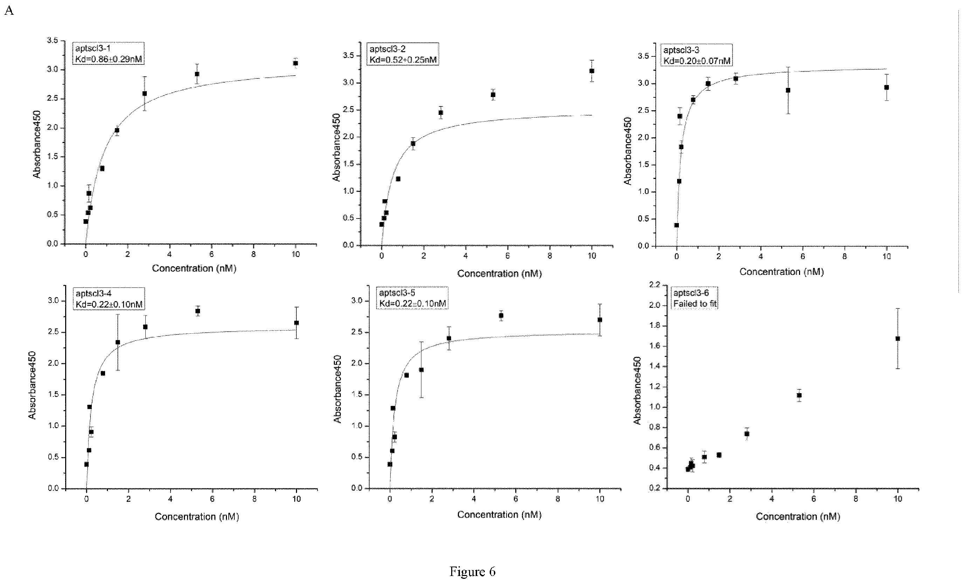

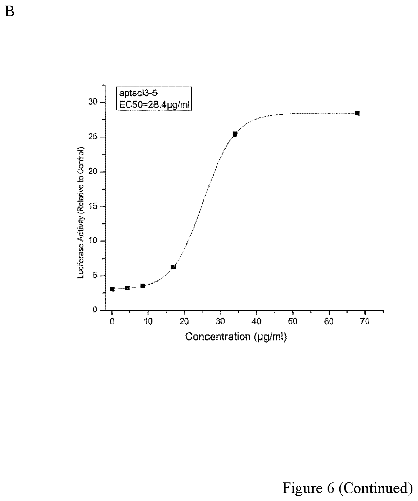

Figure 6. Characterization of aptscl 3 truncations to sclerostin. (A) Truncations aptscl 3-1, -2, -3, -4 and -5 remained high affinity to sclerostin, while aptscl 3-6 showed low binding ability to sclerostin and failed to fit for the affinity analysis; (B) Truncation aptscl 3-5 which remained high binding affinity reserved high inhibition potency to sclerostin's antagonistic effect on Wnt signaling pathway in cells (EC50=28.4 µg/ml).

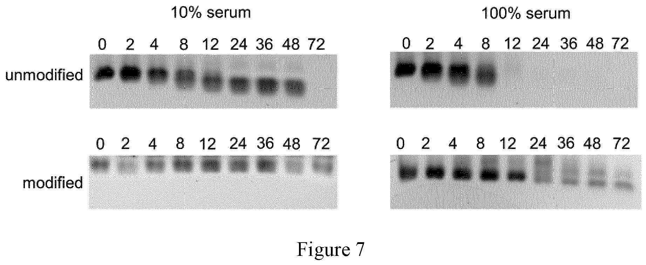

Figure 7. The serum stability assessment of modified aptscl 56 compared to unmodified aptscl 56. All aptamers were treated with 10% and 100% mouse serum from 0 to 72 hours. Nearly all unmodified aptscl56 was degraded 48 h after incubation in 10% mouse serum. The 2'-OMe and 3' idT modified aptscl 56 could last 48 h in 10% mouse serum. In 100% serum, the unmodified aptscl 56 was rapidly and fully degraded after 8 h. At 72 h, a small amount of modified aptamer was still remained.

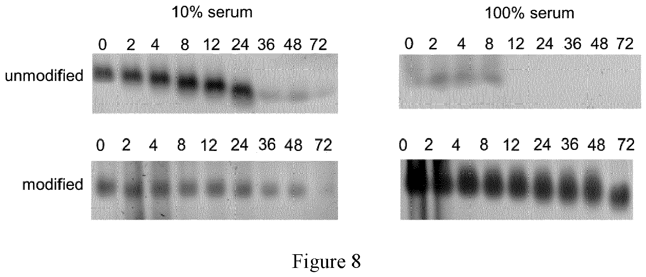

Figure 8. The serum stability assessment of modified aptscl 3-5 compared to unmodified aptscl 3-5. All aptamers were treated with 10% and 100% mouse serum from 0 to 72 hours. Aptscl 3-5 was degraded after 24 h incubation in 10% mouse serum. The 2'-OMe and 3' idT modified aptscl 3-5 could last 48 h in 10% mouse serum. In 100% serum, the unmodified aptscl 3-5 was rapidly and fully degraded after 8 h, while the modified aptscl 3-5 could remain the integrity after 72 h.

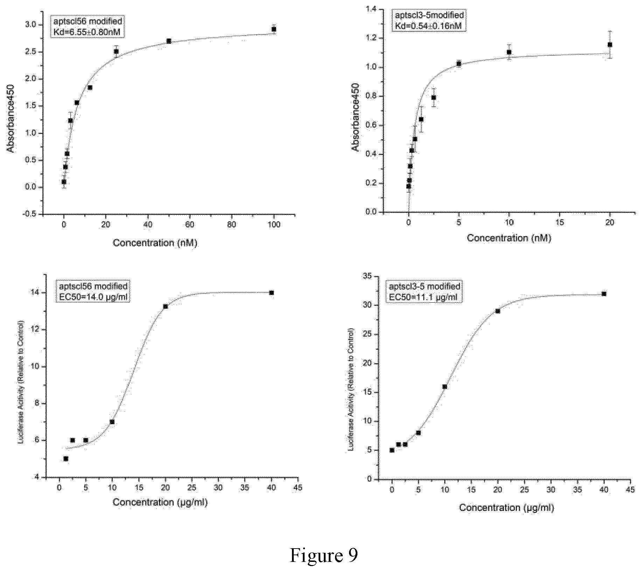

Figure 9. Affinity and inhibition potency of chemically modified aptscl 56 and aptscl 3-5. With chemical modifications, both aptscl56 and aptscl 3-5 remained the high affinity and in vitro inhibition potency to sclerostin.

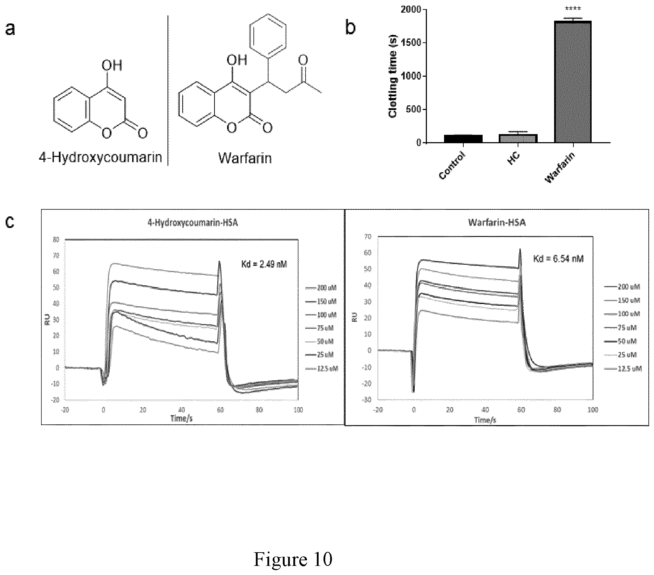

Figure 10 shows a comparison of the anticoagulant effects and serum albumin affinity of hydroxycoumarin and warfarin. a. The structures of 4-hydroxycoumarin and warfarin. b. Investigation of the blood coagulation time of mice with 4-hydroxycoumarin and warfarin. c. Investigation of the affinity of 4-hydroxycoumarin and warfarin to serum albumin.

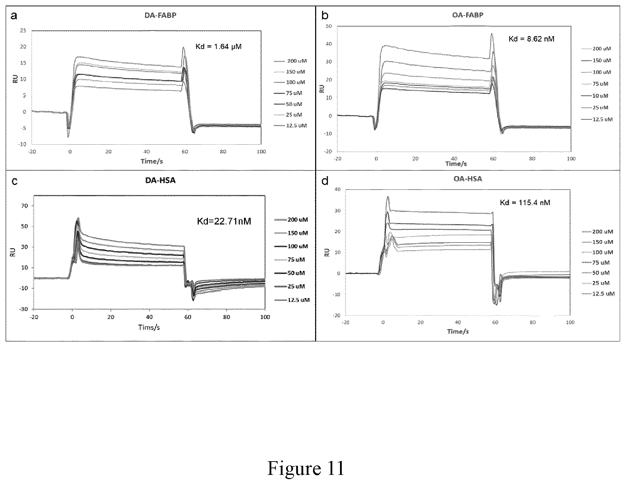

Figure 11 shows a comparison of the affinity of dodecanedioic acid and octadecanedioic acid to fatty acid binding protein and serum albumin. a. Investigation of the affinity between dodecanedioic acid and fatty acid binding protein. b. Investigation of the affinity between octadecanedioic acid and fatty acid binding protein. c. Investigation of the affinity between dodecanedioic acid and serum albumin. d. Investigation of the affinity between octadecanedioic acid and serum albumin.

### Detailed description of the invention

Unless indicated or defined otherwise, all terms used have their usual meaning in the art, which will be clear to the skilled person. Reference is for example made to the standard handbooks, such as Sambrook et al, "Molecular Cloning: A Laboratory Manual"; Lewin, "Genes IV"; and Roitt et al., "Immunology" (8th Ed.), as well as to the general background art cited herein. Furthermore, unless indicated otherwise, all methods, steps, techniques and manipulations that are not specifically described in detail can be performed and have been performed in a manner known per se, as will be clear to the skilled person. Reference is for example again made to the standard handbooks, to the general background art referred to above and to the further references cited therein.

### Definitions

As used herein, the term "nucleotide" refers to a ribonucleotide or a deoxyribonucleotide, or a modified form thereof, as well as an analog thereof. Nucleotides include species that include purines (e.g., adenine, hypoxanthine, guanine, and their derivatives and analogs) as well as pyrimidines (e.g., cytosine, uracil, thymine, and their derivatives and analogs).

As used herein, "nucleic acid," "oligonucleotide," and "polynucleotide" are used interchangeably to refer to a polymer of nucleotides and include DNA, RNA, DNA/RNA hybrids and modifications of these kinds of nucleic acids, oligonucleotides and polynucleotides, wherein the attachment of various entities or moieties to the nucleotide units at any position are included. The terms "polynucleotide," "oligonucleotide," and "nucleic acid" include double- or single- stranded molecules. Nucleic acid, oligonucleotide, and polynucleotide are broader terms than the term aptamer and, thus, the terms nucleic acid, oligonucleotide, and polynucleotide include aptamers but are not limited to aptamers.

As used herein, "aptamer" refers to a non-naturally occurring nucleic acid that has a desirable action on a target molecule. A desirable action includes, but is not limited to, binding of the target, catalytically changing the target, reacting with the target in a way that modifies or alters the target or the functional activity of the target, covalently attaching to the target, and facilitating the reaction between the target and another molecule. In one embodiment, the action is specific binding affinity for a target molecule (such as, sclerostin), such target molecule being a three dimensional chemical structure other than a polynucleotide that binds to the nucleic acid ligand through a mechanism which is independent of Watson/Crick base pairing or triple helix formation, wherein the aptamer is not a nucleic acid having the known physiological function of being bound by the target molecule. In this context, the "specific binding affinity" of an aptamer for its target (such as, sclerostin) means that the aptamer binds to its target generally with a much higher degree of affinity than it binds to other, non-target, components in a mixture or sample.

Sequence "identity" has an art-recognized meaning and the percentage of sequence identity between two nucleic acid or polypeptide molecules or regions can be calculated using published techniques. Sequence identity can be measured along the full length of a polynucleotide or polypeptide or along a region of the molecule. (See, e.g.: Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). While there exist a number of methods to measure identity between two polynucleotide or polypeptides, the term "identity" is well known to skilled artisans (Carrillo, H. & Lipman, D., SIAM J Applied Math 48:1073 (1988)). One example of an algorithm that is suitable for determining percent sequence identity is the algorithm used in the basic local alignment search tool (hereinafter "BLAST"), see, e.g. Altschul et al, J. Mol. Biol. 215:403-410, 1990 and Altschul et al, Nucleic Acids Res., 15:3389-3402, 1997. Software for performing BLAST analyses is publicly available through the National Center for Biotechnology Information (hereinafter "NCBI"). The default parameters used in determining sequence identity using the software available from NCBI, e.g., BLASTN (for nucleotide sequences) are described in McGinnis et al, Nucleic Acids Res., 32:W20-W25, 2004.

### Nucleic acid molecule conjugate with extended in vivo half-life

Fatty acid modification (conjugation) is another means of extending the in vivo half-life of drug molecules. However, fatty acid modification is generally only applied to proteins or peptides, and there are no reports of its application to nucleic acid molecules. Furthermore, fatty acids are lipid-soluble, whereas nucleic acid molecules are water-soluble, and thus one skilled in the art would expect that they are difficult to conjugate. In contrast, the present inventors surprisingly found that the in vivo half-life of a nucleic acid molecule, such as an aptamer, can be significantly extended by conjugating a nucleic acid molecule, such as an aptamer, to a fatty acid, thereby improving its clinical availability.

Furthermore, the inventors surprisingly found that coumarin derivatives such as 4-hydroxycoumarin have high affinity to HSA, and their combination with fatty acids such as dodecanedioic acid can synergistically extend the in vivo half-life of nucleic acid molecules conjugated thereto.

Accordingly, in one aspect, the present invention provides a nucleic acid molecule conjugate with extended in vivo half-life, wherein the nucleic acid molecule is conjugated to a fatty acid and/or a coumarin derivative.

In another aspect, the invention provides a method of extending the in vivo half-life of a nucleic acid molecule, comprising conjugating said nucleic acid molecule to a fatty acid and/or a coumarin derivative.

In some embodiments, the fatty acids include, but are not limited to, palmitic acid (PA), dodecanedioic acid, tetradecanedioic acid, hexadecanedioic acid, stearic acid (SA), octadecanedioic acid, lauric acid, acid, eicosapentaenoic acid (EPA), docosahexaenoic acid (DHA), arachidonic acid (ARA), etc. In some preferred embodiments, the fatty acid is palmitic acid. In some other preferred embodiments, the fatty acid is octadecanedioic acid. In some more preferred embodiments, the fatty acid is dodecanedioic acid.

In some embodiments, the coumarin derivatives include, but are not limited to 4-hydroxycoumarin, 3-acetyl-6-carboxycoumarin, warfarin, (2-oxo-2H-chromen-3-yl)acetic acid, [(8-acetyl-4-methyl-2-oxo-2H-chromen-7-yl)oxy]acetic acid, coumarin-3-carboxylic acid, N-(4-methyl-7-coumarin) oxalamide, 7-(carboxymethyl )-4-methylcoumarin, 7-methoxycoumarin-3-carboxylic acid, 6-methoxy-2-oxo-2H-chromene-3-carboxylic acid. In some preferred embodiments, the coumarin derivative is 4-hydroxycoumarin.

In some embodiments, the fatty acid, such as dodecanedioic acid, is conjugated to the 5' end of the nucleic acid molecule. In some embodiments, the coumarin derivative, such as 4-hydroxycoumarin, is conjugated to the 5' end of the nucleic acid molecule. In some embodiments, the fatty acid, such as dodecanedioic acid, and the coumarin derivative, such as 4-hydroxycoumarin, are conjugated to the 5' end of the nucleic acid molecule.

In some embodiments, the fatty acid, such as dodecanedioic acid, is conjugated to the nucleic acid molecule through a linker. In some embodiments, the coumarin derivative, such as 4-hydroxycoumarin, is conjugated to the nucleic acid molecule via a linker. In some embodiments, the fatty acid, such as dodecanedioic acid, and the coumarin derivative, such as 4-hydroxycoumarin, are conjugated to the nucleic acid molecule through a linker.

In some embodiments, the nucleic acid molecule is a DNA molecule. In some embodiments, the nucleic acid molecule is an RNA molecule. In some embodiments, the nucleic acid molecule is a DNA/RNA hybrid molecule. In some embodiments, the nucleic acid molecule is double-stranded. In some embodiments, the nucleic acid molecule is single-stranded. In some embodiments, the nucleic acid molecule contains a single-stranded portion and a double-stranded portion. In some embodiments, the nucleic acid molecule is an oligonucleotide.

In some embodiments, the nucleic acid molecule is about 1 to about 500 bp/nt in length. In some embodiments, the nucleic acid molecule is about 1 to about 250 bp/nt, about 1 to about 200 bp/nt, about 1 to about 150 bp/nt, about 1 to about 100 bp/nt, about 1 to about 50 bp /nt, about 1 to about 30bp/nt, about 1 to about 20bp/nt, about 1 to about 15bp/nt, about 1 to about 10bp/nt in length. In some embodiments, the nucleic acid molecule is about 500 bp/nt, about 250 bp/nt, about 200 bp/nt, about 150 bp/nt, about 100 bp/nt, about 90 bp/nt, about 80 bp/nt, about 70 bp /nt, about 60bp/nt, about 50bp/nt, about 40bp/nt, about 30bp/nt, about 20bp/nt, about 10bp/nt in length.

In some embodiments, the nucleic acid molecule can be an aptamer, siRNA, antisense RNA, shRNA, etc. In some preferred embodiments, the nucleic acid molecule is an aptamer.

In some embodiments, the nucleic acid molecule of the present invention may also include one or more modifications. For example, the modification is a modification that confers enhanced nuclease resistance to the nucleic acid molecule.

The modification includes, for example, 3' and 5' modification, such as 3' and 5' capping. In some embodiments, the nucleic acid molecule is capped with inverted deoxythymidine at the 3' end, that is, a 3' inverted deoxythymidine (3' idT) modification.

The modification may also include the substitution of one or more naturally occurring nucleotides with modified nucleotides. For example, the modified nucleotides include, but are not limited to, 2'-fluoro, 2'-methoxyethyl, 2'-methoxy and/or 2' allyloxy modified nucleotides (i.e., the 2'-position hydroxyl group of the ribose is substituted by fluorine, methoxyethyl, methoxy or allyloxy, etc.). The modified nucleotides may also include C-5 modified pyrimidines. The term "C-5 modified pyrimidine" refers to a pyrimidine with a modification at the C-5 position. C-5 modified pyrimidines can enhance the nuclease resistance of oligonucleotides, and are known in the art. For example, reference can be made to PCT Application WO 2011/130195 and references cited therein. In some preferred embodiments, the modification is a 2'-methoxy (2'-OMe) modification. In some embodiments, the modification, such as 2'-methoxy (2'-OMe) modification, is made to one or more nucleotides, for example 4 nucleotides, at the 5'and/or 3'end of the nucleic acid molecule.

The modifications also include internucleotide modifications, such as those internucleotide modifications with uncharged bonds (such as methyl phosphonate, phosphotriester, phosphoamine ester, carbamate, etc.) and those internucleotide modifications with charged bonds (such as phosphorothioate, phosphorodithioate, etc.), internucleotide modifications with intercalating agents (such as acridine, psoralen, etc.), internucleotide modifications containing chelating agents (such as metals, radioactive metals, boron, oxidizing metals, etc.), internucleotide modifications containing alkylating agents, and internucleotide modifications with modified bonds (for example, alpha anomeric nucleic acid, etc.).

In some embodiments, the nucleic acid molecule may comprise a combination of modifications described above. For example, the aptamer may comprise a 2'-methoxy (2'-OMe) modification and/or a 3' inverse deoxythymidine (3'idT) modification.

In some embodiments, the in vivo half-life of the nucleic acid molecule conjugate of the invention is at least 2 times, at least 5 times, at least 10 times, at least 25 times, at least 50 times, at least 100 times, at least 200 times or more as compared to a corresponding nucleic acid molecule that does not comprise the conjugated fatty acid and/or coumarin derivative.

### Aptamer conjugate with extended in vivo half-life

Based on the protein-SELEX technology, the present inventors used sclerostin as the target protein for positive screening and unrelated proteins for negative screening, and finally selected aptamers that specifically bind to sclerostin with high affinity. The sclerostin described herein is preferably human sclerostin, for example, the sclerostin whose amino acid sequence is shown in SEQ ID NO:18.

Exemplary human sclerostin amino acid sequence:

QGWQAFKNDATEIIPELGEYPEPPPELENNKTMNRAENGGRPPHHP
FETKDVSEYSCRELHFTRYVTDGPCRSAKPVTELVCSGQCGPARLLPNAI
GRGKWWRPSGPDFRCIPDRYRAQRVQLLCPGGEAPRARKVRLVASCKC

KRLTRFHNQSELKDFGTEAARPQKGRKPRPRARSAKANQAELENAY
(SEQ ID NO:18).

In one aspect, the present invention provides an aptamer conjugate against sclerostin, which comprises an aptamer against sclerostin conjugated to a fatty acid and/or a coumarin derivative,
wherein the aptamer comprises a nucleotide sequence having at least about 90% identity, at least about 91% identity, at least about 90% identity, about 92% identity, at least about 93% identity, at least about 94% identity, at least about 95% identity, at least about 96% identity, at least about 97% identity, at least about 98% identity, or at least about 99% identity to any one of SEQ ID NOs:1-17, or the aptamer comprises at least 30, at least 35, at least 40, at least 45, at least 50, or more consecutive nucleotides within any one of SEQ ID NOs: 1-17. In some embodiments, the aptamer specifically binds to sclerostin. In some preferred embodiments, the aptamer comprises a nucleotide sequence of any one of SEQ ID NOs: 1-17 and 19-25, and more preferably, the aptamer comprises a nucleotide sequence of any one of SEQ ID NOs: 1, 3, 10, 19-23 or 25.

In one aspect, the present invention provides an aptamer conjugate against Dickkopf-1(DKK1), which comprises an aptamer against DKK1 conjugated to a fatty acid and/or a coumarin derivative,
wherein the aptamer comprises a nucleotide sequence having at least about 90% identity, at least about 91% identity, at least about 90% identity, about 92% identity, at least about 93% identity, at least about 94% identity, at least about 95% identity, at least about 96% identity, at least about 97% identity, at least about 98% identity, or at least about 99% identity to any one of SEQ ID NO: 26, or the aptamer comprises at least 30, at least 35, at least 40, at least 45, at least 50, or more consecutive nucleotides within any one of SEQ ID NO: 26. In some embodiments, the aptamer specifically binds to DKK1. In some preferred embodiments, the aptamer comprises a nucleotide sequence of SEQ ID NO: 26.

In some embodiments, the fatty acids include, but are not limited to, palmitic acid (PA), dodecanedioic acid, tetradecanedioic acid, hexadecanedioic acid, stearic acid (SA), octadecanedioic acid, lauric acid, acid, eicosapentaenoic acid (EPA), docosahexaenoic acid (DHA), arachidonic acid (ARA), etc. In some preferred embodiments, the fatty acid is palmitic acid. In some other preferred embodiments, the fatty acid is octadecanedioic acid. In some more preferred embodiments, the fatty acid is dodecanedioic acid.

In some embodiments, the coumarin derivatives include, but are not limited to 4-hydroxycoumarin, 3-acetyl-6-carboxycoumarin, warfarin, (2-oxo-2H-chromen-3-yl)acetic acid, [(8-acetyl-4-methyl-2-oxo-2H-chromen-7-yl)oxy]acetic acid, coumarin-3-carboxylic acid, N-(4-methyl-7-coumarin) oxalamide, 7-(carboxymethyl )-4-methylcoumarin, 7-methoxycoumarin-3-carboxylic acid, 6-methoxy-2-oxo-2H-chromene-3-carboxylic acid. In some preferred embodiments, the coumarin derivative is 4-hydroxycoumarin.

In some embodiments, the fatty acid, such as dodecanedioic acid, is conjugated to the 5' end of the aptamer. In some embodiments, the coumarin derivative, such as 4-hydroxycoumarin, is conjugated to the 5' end of the aptamer. In some embodiments, the fatty acid, such as dodecanedioic acid, and the coumarin derivative, such as 4-hydroxycoumarin, are conjugated to the 5' end of the aptamer.

In some embodiments, the fatty acid, such as dodecanedioic acid, is conjugated to the aptamer through a linker. In some embodiments, the coumarin derivative, such as 4-hydroxycoumarin, is conjugated to the aptamer via a linker. In some embodiments, the fatty acid, such as dodecanedioic acid, and the coumarin derivative, such as 4-hydroxycoumarin, are conjugated to the aptamer through a linker.

In some embodiments, the aptamer or aptamer conjugate against sclerostin of the present invention has a Kd (dissociation constant) to sclerostin of less than 100 nM, preferably less than 50 nM, preferably less than 40 nM, preferably less than 30 nM, preferably less than 20 nM, preferably less than 10 nM or less. The Kd is measured, for example, by enzyme-linked oligonucleotide assay (ELONA).

In some embodiments, the aptamer or aptamer conjugate against sclerostin of the present invention inhibits the biological activity of sclerostin. "Inhibition" means that the biological activity of sclerostin is reduced in the presence of the aptamer or aptamer conjugate compared with the absence of the aptamer, for example, reduced by at least about 10%, at least about 20%, at least about 30%, at least about 40%, at least about 50%, at least about 60%, at least about 70%, at least about 80%, even at least about 90%.

As used herein, the term "biological activity" refers to an effect on one or more cellular or extracellular processes, which can affect physiological or pathophysiological processes. The biological activities of sclerostin include, but are not limited to, antagonizing the Wnt signaling pathway.

In some embodiments, the aptamer or aptamer conjugate against sclerostin of the present invention can inhibit the antagonistic effect of sclerostin on the Wnt signaling pathway. For example, the aptamer or aptamer conjugate against sclerostin of the present invention can block the antagonistic effect of sclerostin in a cell-based Wnt signaling assay.

In some embodiments, the aptamer or aptamer conjugate against sclerostin of the present invention inhibits the biological activity of sclerostin, such as inhibits the antagonistic effect of sclerostin on the Wnt signaling pathway with a EC50 value of less than 100 µg/ml, preferably less than 50 µg/ml, preferably less than 40 µg/ml, preferably less than 30 µg/ml, preferably less than 20 µg/ml, preferably less than 10 µg/ml or less. In some embodiments, the EC50 value is determined in vitro by a TOP-Wnt-induced luciferase reporter gene assay in osteoblasts.

In some embodiments, the aptamer of the present invention may also include one or more modifications. For example, the modification is a modification that confers enhanced nuclease resistance to the aptamer and/or enhances the in vivo half-life of the aptamer.

The modification includes, for example, 3' and 5' modification, such as 3' and 5' capping. In some embodiments, the aptamer is capped with inverted deoxythymidine at the 3' end, that is, a 3' inverted deoxythymidine (3' idT) modification.

The modification may also include the substitution of one or more naturally occurring nucleotides with modified nucleotides. For example, the modified nucleotides include, but are not limited to, 2'-fluoro, 2'-methoxyethyl, 2'-methoxy and/or 2' allyloxy modified nucleotides (i.e., the 2'-position hydroxyl group of the ribose is substituted by fluorine, methoxyethyl, methoxy or allyloxy, etc.). The modified nucleotides may also include C-5 modified pyrimidines. The term "C-5 modified pyrimidine" refers to a pyrimidine with a modification at the C-5 position. C-5 modified pyrimidines can enhance the nuclease resistance of oligonucleotides, and are known in the art. For example, reference can be made to PCT Application WO 2011/130195 and references cited therein. In some preferred embodiments, the modification is a 2'-methoxy (2'-OMe) modification. In some embodiments, the modification, such as 2'-methoxy (2'-OMe) modification, is made to one or more nucleotides, for example 4 nucleotides, at the 5'and/or 3'end of the aptamer.

The modifications also include internucleotide modifications, such as those internucleotide modifications with uncharged bonds (such as methyl phosphonate, phosphotriester, phosphoamine ester, carbamate, etc.) and those internucleotide modifications with charged bonds (such as phosphorothioate, phosphorodithioate, etc.), internucleotide modifications with intercalating agents (such as acridine, psoralen, etc.), internucleotide modifications containing chelating agents (such as metals, radioactive metals, boron, oxidizing metals, etc.), internucleotide modifications containing alkylating agents, and internucleotide modifications with modified bonds (for example, alpha anomeric nucleic acid, etc.).

In some embodiments, the aptamer may comprise a combination of various modifications described above. For example, the aptamer may include 2'-methoxy (2'-OMe) modification and/or 3' inverted deoxythymidine (3' idT) modification.

In some specific embodiments, the aptamer conjugate comprises the following sequence structure: FA-linker-C(OMe)G(OMe)G(OMe)G(OMe)GTGTGGGTTCGTCGTTAGCTT GATTTGGCAGCU(OMe)G(OMe)C( OMe)C(OMe)-idT, where FA represents a fatty acid, (OMe) represents the 2'-methoxy (2'-OMe) modification of the corresponding nucleotide, and idT represents a 3' reverse deoxythymidine modification. In some specific embodiments, the fatty acid is palmitic acid (PA). In some specific embodiments, the fatty acid is octadecanedioic acid. In some preferred embodiments, the fatty acid is dodecanedioic acid (DA).

In some specific embodiments, the aptamer conjugate comprises the following sequence structure: fatty acid/coumarin derivative-linker-C(OMe)G(OMe)G(OMe)G(OMe)GTGTGGGTTCGTCGTTA GCTTGATTTGGCAGCU(OMe) G(OMe)C(OMe)C(OMe)-idT, where (OMe) represents the 2'-methoxy (2'-OMe) modification of the corresponding nucleotide, and idT represents the 3' reverse deoxythymidine modification. In some preferred embodiments, the fatty acid is dodecanedioic acid (DA). In some preferred embodiments, the coumarin derivative is 4-hydroxycoumarin.

### Methods for preparing nucleic acid molecule conjugate

In one aspect, the invention provides a method for preparing a nucleic acid molecule conjugate of the invention, wherein the nucleic acid molecule is conjugated to a fatty acid. The method includes activating the carboxyl group of the fatty acid to form an active ester structure, which then reacts with the nucleic acid molecular structure selected from S1 to S5 below,

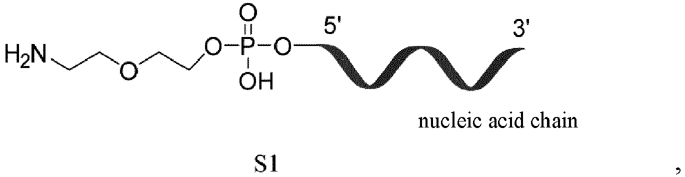

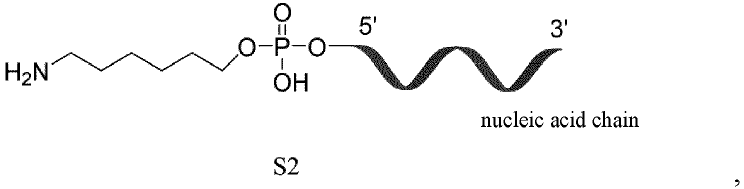

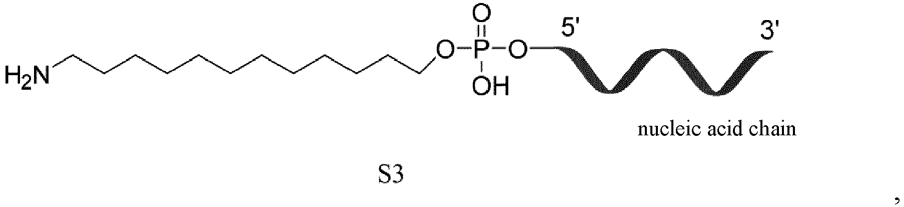

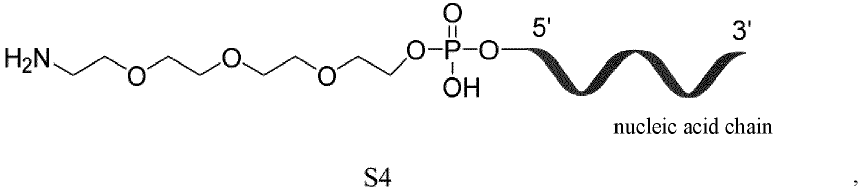

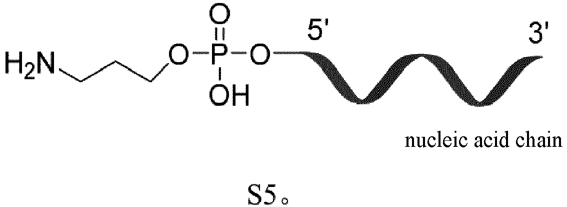

The nucleic acid molecules such as aptamers and fatty acids are as defined above. In the nucleic acid molecular structure of S1 to S5, the functional group at the 5' end is inserted using commercial amino monomers through conventional nucleic acid solid-phase synthesis conditions.

In one aspect, the invention provides a method for preparing a nucleic acid molecule conjugate of the invention, wherein the nucleic acid molecule is conjugated to a fatty acid and a coumarin derivative, the method comprises allowing the fatty acid or a chemical intermediate thereof suitable for reaction to react with the amino group of the nucleic acid molecular structure of S6, and allowing the coumarin derivative or a chemical intermediate thereof suitable for reaction to react with the sulfhydryl group of the nucleic acid molecular structure of S6,

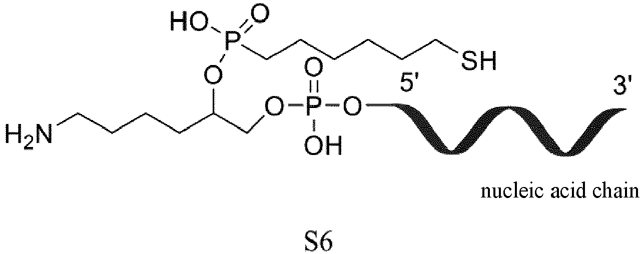

The nucleic acid molecules such as aptamers, fatty acids and coumarin derivatives are as defined above.

### Treatment of diseases

In another aspect, the present invention provides a method for treating diseases by the aptamer conjugate against sclerostin of the present invention, the method comprises administering a therapeutically effective amount of the aptamer conjugate against sclerostin of the present invention to a subject in need thereof.

The diseases treated by the aptamer conjugate against sclerostin of the present invention are, for example, sclerostin-related diseases, such as sclerostin-mediated diseases.

As used herein, "a sclerostin-related disease" includes diseases in which bone mineral density (BMD) is abnormally and/or pathologically low relative to healthy subjects. Diseases characterized by low BMD and/or bone fragility include but are not limited to primary and secondary osteoporosis, osteopenia, osteomalacia, osteogenesis imperfecta (OI), avascular necrosis (osteonecrosis), fractures and implant healing (dental implants and hip implants), bone loss due to other diseases (e.g., associated with HIV infection, cancers, or arthritis). Other "sclerostin-related diseases" include but are not limited to Hypophosphatemic rickets, rheumatoid arthritis, osteoarthritis, arthritis, and osteolytic lesions.

As used herein, "a sclerostin-related disease" includes sclerostin-related cancers, such as myeloma (e.g., multiple myeloma with osteolytic lesions), breast cancer (such as, triple negative breast cancer), colon cancer, melanoma, hepatocellular cancer, epithelial cancer, esophageal cancer, brain cancer, lung cancer, prostate cancer, or pancreatic cancer, as well as any metastases thereof.

A "sclerostin-related disease" can also include renal and cardiovascular conditions, due at least to sclerostin's expression in the kidney and cardiovasculature. Said diseases include but are not limited to such renal disorders as glomerular diseases (e.g., acute and chronic glomerulonephritis, rapidly progressive glomerulonephritis, nephrotic syndrome, focal proliferative glomerulonephritis, glomerular lesions associated with systemic disease, such as systemic lupus erythematosus, Goodpasture's syndrome, multiple myeloma, diabetes, polycystic kidney disease, neoplasia, sickle cell disease, and chronic inflammatory diseases), tubular diseases (e.g., acute tubular necrosis and acute renal failure, polycystic renal diseasemedullary sponge kidney, medullary cystic disease, nephrogenic diabetes, and renal tubular acidosis), tubulointerstitial diseases (e.g., pyelonephritis, drug and toxin induced tubulointerstitial nephritis, hypercalcemic nephropathy, and hypokalemic nephropathy) acute and rapidly progressive renal failure, chronic renal failure, nephrolithiasis, gout, vascular diseases (e.g., hypertension and nephrosclerosis, microangiopathic hemolytic anemia, atheroembolic renal disease, diffuse cortical necrosis, and renal infarcts), or tumors (e.g., renal cell carcinoma and nephroblastoma).

Said sclerostin-related diseases also include but are not limited to such cardiovascular disorders as ischemic heart disease (e.g., angina pectoris, myocardial infarction, and chronic ischemic heart disease), hypertensive heart disease, pulmonary heart disease, valvular heart disease (e.g., rheumatic fever and rheumatic heart disease, endocarditis, mitral valve prolapse, and aortic valve stenosis), congenital heart disease (e.g., valvular and vascular obstructive lesions, atrial or ventricular septal defect, and patent ductus arteriosus), or myocardial disease (e.g., myocarditis, congestive cardiomyopathy, and hypertrophic cariomyopathy).

In another aspect, the present invention provides a method for treating diseases by the aptamer conjugate against DKK1 of the present invention, the method comprises administering a therapeutically effective amount of the aptamer conjugate against DKK1 of the present invention to a subject in need thereof.

The diseases treated by the aptamer conjugate against DKK1 of the present invention are, for example, DKK1-related diseases, such as DKK1-mediated diseases.

As used herein, "a DKK1-related disease" includes sclerostin-related cancers, such as myeloma (e.g., multiple myeloma with osteolytic lesions, Hilar cholangiocarcinoma multiple myeloma), breast cancer, colon cancer, melanoma, hepatocellular cancer, epithelial cancer, esophageal cancer, brain cancer, lung cancer, prostate cancer, or pancreatic cancer, as well as any metastases thereof.

In some embodiments, the DKK1-related disease is selected from the group consisting of osteoporosis, osteopenia, osteomalacia, osteogenesis imperfecta (OI), avascular osteonecrosis, rheumatoid arthritis, fractures, osteoarthritis, and myeloma.

The subject can be any animal (domesticated, domestic animal or wild), including but not limited to cats, dogs, horses, pigs, and cows, and human subjects are preferred. As used herein, the terms patient, individual, and subject can be used interchangeably.

The subject can be a male or a female. Preferably, the human subject is at risk of fracture, more preferably the human subject is at risk of osteoporosis or suffers from osteoporosis. The human subject is preferably a female, and more preferably a female at risk of post-menopausal osteoporosis or suffering from post-menopausal osteoporosis. It is expected that the method of the present invention can be beneficial to subjects at any stage of osteoporosis.

As used herein, "treating" an individual suffering from a disease or disease condition means that the individual' s symptoms are partially or completely alleviated, or remain unchanged after treatment. Thus, treatment includes prevention, treatment and/or cure. Prevention refers to prevention of a potential disease and/or prevention of worsening of symptoms or disease progression.

As used herein, "therapeutically effective amount" or "therapeutically effective dose" refers to an amount of a substance, compound, material, or composition comprising a compound that is at least sufficient to produce a therapeutic effect after administration to a subject. Thus, it is the amount necessary to prevent, cure, ameliorate, arrest or partially arrest the symptoms of the disease or condition. As used herein, "therapeutic effect" means an effect resulting from treatment of an individual that alters, generally ameliorates or alleviates the symptoms of the disease or disease condition, or cures the disease or disease condition.

The dosage regimen of the aptamer conjugate is selected according to a variety of factors, including, for example, the type, species, age, weight, gender, and medical condition of the patient; the severity of the condition to be treated; the route of administration; the kidney function and liver function of the patient; and the specific aptamer conjugate or salt thereof as used. Ordinary skilled doctors can easily determine and specify the effective amount of the composition required to prevent, combat, or inhibit the progression of the condition.

Typically, the dosage regimen of the aptamer conjugate is about 1 µg/kg body weight to about 100 mg/kg body weight per day.

An exemplary treatment regime entails administration once daily, once every two days, once per week, twice per week, once every two weeks, once every three weeks, once every four weeks, once a month, once every 3 months or once every three to 6 months, or with a short administration interval at the beginning (such as once per week to once every three weeks), and then an extended interval later (such as once a month to once every three to 6 months). The frequency and interval of administration can be determined by those skilled in the art according to the pharmacokinetic parameters of the aptamer conjugate.

### Pharmaceutical composition

In another aspect, the present invention also provides a pharmaceutical composition comprising at least one aptamer conjugate against sclerostin of the present invention, and a pharmaceutically acceptable carrier or excipient. Said pharmaceutical composition is used for treating, for example, sclerostin-related diseases.

In another aspect, the present invention also provides a pharmaceutical composition comprising at least one aptamer conjugate against DKK1 of the present invention, and a pharmaceutically acceptable carrier or excipient. Said pharmaceutical composition is used for treating, for example, DKK1-related diseases.

The aptamer conjugate described herein can be utilized in any pharmaceutically acceptable dosage form, including but not limited to injectable dosage forms, liquid dispersions, gels, aerosols, ointments, creams, lyophilized formulations, dry powders, tablets, capsules, controlled release formulations, fast melt formulations, delayed release formulations, extended release formulations, pulsatile release formulations, mixed immediate release and controlled release formulations, etc. Specifically, the aptamers described herein can be formulated: (a) for administration selected from any of oral, pulmonary, intravenous, intra-arterial, intrathecal, intraarticular, rectal, ophthalmic, colonic, parenteral, intracisternal, intravaginal, intraperitoneal, local, buccal, nasal, and topical administration; (b) into a dosage form selected from any of liquid dispersions, gels, aerosols, ointments, creams, tablets, sachets and capsules; (c) into a dosage form selected from any of lyophilized formulations, dry powders, fast melt formulations, controlled release formulations, delayed release formulations, extended release formulations, pulsatile release formulations, and mixed immediate release and controlled release formulations; or (d) any combination thereof.

Solutions or suspensions used for parenteral, intradermal, or subcutaneous application can comprise one or more of the following components: (1) a sterile diluent such as water for injection, saline solution, fixed oils, polyethylene glycols, glycerin, propylene glycol or other synthetic solvents; (2) antibacterial agents such as benzyl alcohol or methyl parabens; (3) antioxidants such as ascorbic acid or sodium bisulfite; (4) chelating agents such as ethylenediaminetetraacetic acid; (5) buffers such as acetates, citrates or phosphates; and (6) agents for the adjustment of tonicity such as sodium chloride or dextrose. The pH can be adjusted with acids or bases, such as hydrochloric acid or sodium hydroxide. A parenteral preparation can be enclosed in ampoules, disposable syringes or multiple dose vials made of glass or plastic.

Pharmaceutical compositions suitable for injectable use may include sterile aqueous solutions (where water soluble) or dispersions and sterile powders for the extemporaneous preparation of sterile injectable solutions or dispersion. For intravenous administration, suitable carriers include physiological saline, bacteriostatic water, or phosphate buffered saline (PBS). In all cases, the composition should be sterile and should be fluid to the extent that easy syringability exists. The pharmaceutical composition should be stable under the conditions of manufacture and storage and should be preserved against the contaminating action of microorganisms such as bacteria and fungi. The term "stable", as used herein, means remaining in a state or condition that is suitable for administration to a patient.

The carrier can be a solvent or dispersion medium, including, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol or sorbitol, and inorganic salts such as sodium chloride in the composition. Prolonged absorption of the injectable compositions can be brought about by including in the composition an agent which delays absorption, for example, aluminum monostearate and gelatin.

Sterile injectable solutions can be prepared by incorporating the active reagent (e.g., an aptamer conjugate) in the required amount in an appropriate solvent with one or a combination of ingredients enumerated above, as required, followed by filtered sterilization. Generally, dispersions are prepared by incorporating at least one aptamer conjugate into a sterile vehicle that contains a basic dispersion medium and any other required ingredient. In the case of sterile powders for the preparation of sterile injectable solutions, exemplary methods of preparation include vacuum drying and freeze-drying, both of which will yield a powder of the aptamer conjugate plus any additional desired ingredient from a previously sterile-filtered solution thereof.

Oral compositions generally include an inert diluent or an edible carrier. They can be enclosed, for example, in gelatin capsules or compressed into tablets. For the purpose of oral therapeutic administration, the aptamer conjugate can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition.

For administration by inhalation, the compounds are delivered in the form of an aerosol spray from a pressured container or dispenser that contains a suitable propellant, e.g., a gas such as carbon dioxide, a nebulized liquid, or a dry powder from a suitable device. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active reagents are formulated into ointments, salves, gels, or creams as generally known in the art. The reagents can also be prepared in the form of suppositories (e.g., with conventional suppository bases such as cocoa butter and other glycerides) or retention enemas for rectal delivery.

In one embodiment, the aptamer conjugate is formulated for topical administration. As used herein "topical administration" refers to the delivery of an aptamer conjugate to an animal by contacting, directly or otherwise, a formulation comprising the aptamer conjugate to all or a portion of the skin (epidermis) of an animal. The term encompasses several routes of administration including, but not limited to, topical and transdermal. A common requirement for these modes of administration is efficient delivery to the target tissue or stratum. In one aspect, topical administration is used as a means to penetrate the epidermis and dermis and ultimately achieve systemic delivery of the aptamer conjugate. In another aspect, topical administration is used as a means to selectively deliver the aptamer conjugate to the epidermis or dermis of an animal, or to specific strata thereof.

For topical administration, the aptamer conjugate may be formulated into pharmaceutically acceptable ointments, creams, lotions, eye ointments, eye drops, ear drops, impregnated dressings, and aerosols, medicated powders, medicated adhesives, foams, and may contain appropriate conventional additives or excipients, including, for example, preservatives or solvents to assist drug penetration, and emollients in ointments, gels, and creams. Such topical formulations may also contain compatible conventional carriers, for example ethanol or oleyl alcohol for lotions. Such carriers may constitute from about 1% to about 98% by weight of the formulation; more usually, such carriers will constitute up to about 80% by weight of the formulation. Specific formulations for the topical delivery of aptamers are described in the art.

In one embodiment, an aptamer conjugate is prepared with a carrier that will protect against rapid elimination from the body. For example, a controlled release formulation can be used, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

Liposomal suspensions can also be used as pharmaceutically acceptable carriers. These can be prepared according to methods known to those skilled in the art.

Additionally, suspensions of the aptamer conjugate may be prepared as appropriate oily injection suspensions. Suitable lipophilic solvents or vehicles include fatty oils, such as sesame oil, or synthetic fatty acid esters, such as ethyl oleate, triglycerides, or liposomes. Nonlipid polycationic amino polymers may also be used for delivery. Optionally, the suspension may also include suitable stabilizers or agents to increase the solubility of the compounds and allow for the preparation of highly concentrated solutions.

In some cases, it may be especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of an aptamer conjugate calculated to produce the desired therapeutic effect in association with the required pharmaceutical carrier. The specification for the dosage unit forms of the aptamer conjugate described herein are dictated by and directly dependent on the unique characteristics of the particular aptamer conjugate and the particular therapeutic effect to be achieved, and the limitations inherent in the art of compounding such an active agent for the treatment of individuals.

Pharmaceutical compositions comprising at least one aptamer conjugate can include one or more pharmaceutical excipients. Examples of such excipients include, but are not limited to, binding agents, filling agents, lubricating agents, suspending agents, sweeteners, flavoring agents, preservatives, buffers, wetting agents, disintegrants, effervescent agents, and other excipients. Such excipients are known in the art. Exemplary excipients include: (1) binding agents which include various celluloses and cross-linked polyvinylpyrrolidone, microcrystalhne cellulose, such as Avicel PH101 and Avicel PH102, silicified microcrystalhne cellulose (ProSolv SMCC^{™}), gum tragacanth and gelatin; (2) filling agents such as various starches, lactose, lactose monohydrate, and lactose anhydrous; (3) disintegrating agents such as alginic acid, Primogel, corn starch, lightly crosslinked polyvinyl pyrrolidone, potato starch, maize starch, and modified starches, croscarmellose sodium, cross-povidone, sodium starch glycolate, and mixtures thereof; (4) lubricants, including agents that act on the flowability of a powder to be compressed, include magnesium stearate, colloidal silicon dioxide, such as Aerosil 200, talc, stearic acid, calcium stearate, and silica gel; (5) glidants such as colloidal silicon dioxide; (6) preservatives, such as potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quaternary compounds such as benzalkonium chloride; (7) diluents such as pharmaceutically acceptable inert fillers, such as microcrystalhne cellulose, lactose, dibasic calcium phosphate, saccharides, and/or mixtures of any of the foregoing; examples of diluents include microcrystalline cellulose, such as Avicel PH101 and Avicel PH102; lactose such as lactose monohydrate, lactose anhydrous, and Pharmatose DCL21; dibasic calcium phosphate such as Emcompress; mannitol; starch; sorbitol; sucrose; and glucose; (8) sweetening agents, including any natural or artificial sweetener, such as sucrose, saccharin sucrose, xylitol, sodium saccharin, cyclamate, aspartame, and acesulfame; (9) flavoring agents, such as peppermint, methyl salicylate, orange flavoring, Magnasweet (trademark of MAFCO), bubble gum flavor, fruit flavors, and the like; and (10) effervescent agents, including effervescent couples such as an organic acid and a carbonate or bicarbonate.

### Examples

The present invention is further described by the following examples, but the scope of the present invention is not limited to the described examples.

### Example 1. Enrichment and selection of high affinity aptamers against sclerostin

The ssDNA library consists an 18 nt conserved region at each end and a central randomized region. Two ssDNA libraries with different length of randomized sequence were used in this project. The long ssDNA library contains a 40 nt random region (5'-CGTACGGTCGACGCTAGC-(N)₄₀-CACGTGGAGCTCGGATCC-3') and the short ssDNA contains a 25 nt random region (5'-CGTACGGTCGACGCTAGC-(N)₂₅-CACGTGGAGCTCGGATCC-3'). A forward primer (FP: 5'-CGTACGGTCGACGCTAGC-3') and a biotinylated reverse primer (Bio-RP: 5'-biotin-GGATCCGAGCTCCACGTG-3') were synthesized for the amplification of ssDNA during selection. All oligos were purified by HPLC after synthesis.

Protein-SELEX methodology was performed to identify high affinity aptamers (Ellington and Szostak 1990, Tuerk and Gold 1990). 100-30 pmole His₆-sclerostin protein was immobilized on NTA magnetic beads at 4°C for 1h (Murphy, Fuller et al. 2003). 1 nmole ssDNA library was denatured at 95 °C for 5 min and rapidly cooled down to 4 °C followed by incubation with immobilized sclerostin protein at R.T. for 0.5-1 h. Unbound sequences were removed with washing buffer. After washing, the bound DNAs-protein-NTA were collected, resuspended with H₂O/Tween20 and applied to PCR amplification. PCR was performed with unmodified forward primer and 1 Biotinylated reverse primer (step 1: 95 °C 1 min for initial denaturation; step 2: 95 °C 30 s for denaturation, 56 °C 30 s for annealing, 72°C, 30 s for elongation, repeated for 12 cycles; and step 3: 72 °C 5 min for final extension). PCR products were applied to streptavidin magnetic beads through Biotin-streptavidin binding. Single stranded sequences were regenerated by treating with 0.2 M NaOH. Negative selection was performed against other His₆-tagged non-relevant proteins immobilized on the NTA magnetic beads. 20 rounds of SELEX in total was performed for each selection. The DNA pool from the final round was sent for high-throughput Next Generation Sequence (NGS).

Figure 1 shows that the affinity of the DNA pool to sclerostin increases after the 10th and 20th rounds of selection, indicating that high-affinity sclerostin aptamers are enriched by SELEX.

### Example 2. Specificity characterization of the candidate sclerostin aptamers

According to the NGS results, representative aptamers with high occurrence were synthesized for specificity assay. Detailed sequences of these aptamer candidates were listed in Table 1.

For determining the specificity of aptamer candidates to sclerostin, representative aptamer candidates and random sequence (RS) (negative control) were synthesized with N-terminal biotinylated modification and 1 µM of each aptamer/RS were used to determine the specificity to sclerostin using an Enzyme-Linked OligoNucleotide Assay (ELONA). 160 ng purified recombinant human sclerostin was coated to 96-well microtiter plate in 100 µl PBS by incubating at 4 °C overnight. The plate was then blocked with blocking buffer (PBS, 0.1% Tween 20 and 1% BSA) for 1 h at room temperature and washed with SELEX B&W buffer (PBS, 1mM MgCl2, 0.1% Tween 20 and 0.1% BSA) for 4 times. The aptamer candidates were denatured at 95 °C for 10 min and rapidly cooled on ice for 10 min before use. 1 µM biotinylated aptamers were added into each well, then added SELEX B&W buffer to 100 µl and incubated for 45 min at room temperature with continuous gentle shaking. After binding, the plate was washed with SELEX B&W buffer for 4 times to remove non-specific and very weak binding, followed by washing with PBST+0.1% BSA for 4 times. 100 µl streptavidin-HRP/goat anti-human IgG Fc-HRP (1:10000 dilute into PBST+0.1% BSA) was added to each well and incubated for 30/60 min and washed with PBST+0.1% BSA for 4 times. 50 µl TMB was added to each well and incubated for 20 min. The reaction was stopped by adding 50 µl 2 M H2SO4. Absorbance at 450 nm was measured with microplate reader (Stoltenburg, Krafcikova et al. 2016). For determining the binding ability of the aptamer candidates to hepatocytes/PBMCs, the characterization steps were similar with ELONA. 300,000 cells were incubated with each aptamer candidates and the centrifugations were used for washing and separation purposes.

Aptamer candidates identified from both long and short ssDNA libraries showed high selectivity to human sclerostin when comparing the bindings to hepatocytes and PBMCs (Figure 2). Aptamer candidates aptscl 6, 9, 15, 27, 34, 36, 46, 51, 56, 132 and 140 which identified from the long ssDNA library, aptscl 1, 2, 3, 5, 8, 12. 16, 22, 29 and 32 which identified from the short ssDNA library showed high binding specificity to sclerostin and therefore were chosen for the following affinity characterization.

### Example 3. Binding affinity characterization of the candidate sclerostin aptamers

An Enzyme-linked OligoNucleotide Assay (ELONA) was performed to determine the binding affinity of the aptamer candidates to sclerostin (Drolet, Moon-McDermott et al. 1996). Similarly, an Enzyme-linked ImmunoSorbent Assay (ELISA) was performed to determine the binding affinity of anti-sclerostin antibody to human sclerostin (Engvall and Perlmann 1971). 160 ng purified recombinant human sclerostin was coated to 96-well microtiter plate in 100 µl PBS by incubating at 4 °C overnight. The plate was then blocked with blocking buffer (PBS, 0.1% Tween 20 and 1% BSA) for 1 h at room temperature and washed with SELEX B&W buffer (PBS, 1mM MgCl₂, 0.1% Tween 20 and 0.1% BSA) for 4 times. The aptamer candidates were denatured at 95 °C for 10 min and rapidly cooled on ice for 10 min before use. Appropriate concentrations of biotinylated aptamers/antibody were added into each well, then added SELEX B&W buffer to 100 µl and incubated for 45 min at room temperature with continuous gentle shaking. After binding, the plate was washed with SELEX B&W buffer for 4 times to remove non-specific and very weak binding, followed by washing with PBST+0.1% BSA for 4 times. 100 µl streptavidin-HRP/goat anti-human IgG Fc-HRP (1:10000 dilute into PBST+0.1% BSA) was added to each well and incubated for 30/60 min and washed with PBST+0.1% BSA for 4 times. 50 µl TMB was added to each well and incubated for 20 min. The reaction was stopped by adding 50 µl 2 M H₂SO₄. Absorbance at 450 nm was measured with microplate reader (Stoltenburg, Krafcikova et al. 2016). Data was analyzed with Origin software (OriginLab, Northampton, MA). A non-linear curve fitting model Hyperbl was used to plot the binding curve. Equation of Hyperbl model is y = P1*x/(P2 + x) and P2 is the Kd value.

For the aptamer candidates which were identified from ssDNA library containing a 40 nt random region, aptscl 6, 9, 15, 46, 56 and 132 showed high affinity to sclerostin with dissociation constant (Kd) value at nanomolar level (Kd value was 4.2, 3.4, 45.6, 43.1 and 42.2 nM, respectively) (Figure 3). While aptscl 36, 140, 136 and the random sequence (RS) were failed to be fitted. For the aptamer candidates which were identified from ssDNA library containing a 25 nt random region, aptscl 32, 29, 22, 16, 3, 2 and 1 showed higher binding affinity to sclerostin with Kd value at 0.18, 0.28, 0.76, 0.02, 0.04, 0.006 and 0.02 nM, respectively (Figure 4). The random sequence showed low binding ability to sclerostin and failed to be fitted. In comparison, the Kd value of anti-sclerostin antibody to sclerostin was 3.55 nM.

### Example 4. In vitro evaluation of the inhibitory ability of the candidate sclerostin aptamer on the activity of osteosclerosis

To study the inhibition potency of aptamers against sclerostin's antagonistic effect on Wnt signaling, a TOP-Wnt induced luciferase reporter assay was used in osteoblast MC3T3-E1 cells (van Bezooijen, Svensson et al. 2007, Shum, Chan et al. 2011).

MC3T3-E1 cells were seeded in 24-well plates and cells were transfected with corresponding reporter plasmid (100 ng), Wnt3a plasmid (800 ng) and sclerostin plasmid (800 ng) as necessary using FuGENE HD transfection reagent (Promega) in the following day. 10 hours after transfection, culture medium was changed to fresh medium and cells were treated with aptamers/antibody. 24 hours after treatment, each well of cells was lysed with 100 µl passive lysis buffer and 20 µl was taken for analysis. The Luciferase Assay Reagent II and Stop & Glo Reagent were prepared and added automatically by SpectraMax i3x Multi-Mode Detection Platform (Molecular Device) according to the manufacturer's protocol (Promega) and data was analyzed accordingly (Grentzmann, Ingram et al. 1998, McNabb, Reed et al. 2005).

As shown in Figure 5, aptscl 56, aptscl 6, aptscl 3 and anti-sclerostin antibody can effectively inhibit sclerostin's antagonistic effect on Wnt signaling and release the Wnt-induced luciferase activity. The inhibition to sclerostin was dose dependent and the response was stable when the concentrations of aptscl 56 and 6 reached to 25 and 47.4 µg/ml, respectively. While treated with antibody, the response was still not stable with the concentration increasing to 20 mg/ml. Furthermore, the inhibition potency of aptscl 56, aptscl 6 and aptscl 3 was analyzed with non-linear curve fitting. The EC50 for aptscl56, aptscl6 and aptscl 3 was 19.7 µg/ml, 36.8 µg/ml and 18.2 µg/ml, respectively.

**Table 1. Sequences of osteosclerosin aptamer candidates**

| Name | SEQ ID NO | Sequence against sclerostin (5' to 3') | Leng th(nt) | Initial librar y | Kd (nM) | EC₅₀(µg/ ml) |
|---|---|---|---|---|---|---|
| aptsci 56 | 1 | CGGGGTGTGGGTTCGTCGTTAGCTTGAT TTGGCAGCTGCC | 40 | Long | 43.1 | 19.7 |
| aptscl 132 | 2 | CCCAGACGAGACACCTCATGCTTTTCCC CGGGGGAGGGGTAT | 42 | Long | 42.2 | No Inhibition |
| aptsci 6 | 3 | CGTACGGTCGACGCTAGCTGGAAGGGTG GGGGCGGGGGGTCCTCGCCTCGAACGT ACGCACGTGGAGCTCGGATCC | 76 | Long | 4.2 | 36.8 |
| aptscl 9 | 4 | CGTACGGTCGACGCTAGCTGGGGGTAG GGGGACCCTGGCTAGTTAGTCACCGTTT CGACACGTGGAGCTCGGATCC | 76 | Long | 3.4 | No Inhibition |
| aptscl 15 | 5 | CGTACGGTCGACGCTAGCGAGGGGGCC AACTATGCTTAGTGGGGGGGTTGACCGT ATCCACGTGGAGCTCGGATCC | 76 | Long | 4 | No Inhibition |
| aptscl 46 | 6 | CGTACGGTCGACGCTAGCGGGTGGATTA AGGGGGCCCCGTCTGTAGGCGATTGGC GAACACGTGGAGCTCGGATCC | 76 | Long | 45.6 | No Inhibition |
| aptsci 51 | 7 | CGTACGGTCGACGCTAGCTGGGGGTAG GGGGTCCTTGGCTACGGTGATTCGGATG TGACACGTGGAGCTCGGATCC | 76 | Long | 62.2 | No Inhibition |
| aptscl 1 | 8 | CGTACGGTCGACGCTAGCTCTTGTTCATC GATCCTACGCACGTGGAGCTCGGATCC | 56 | Short | 0.02 | No Inhibition |
| aptscl 2 | 9 | CGTACGGTCGACGCTAGCCAGCCAAAGA CAGAGATGCACGTGGAGCTCGGATCC | 54 | Short | 0.006 | No Inhibition |
| aptscl 3 | 10 | CGTACGGTCGACGCTAGCTGTTGTACAT CGCCTTACGCACGTGGAGCTCGGATCC | 55 | Short | 0.04 | 18.2 |
| aptscl 5 | 11 | CGTACGGTCGACGCTAGCTGTTGTTCAT CGACTTGACGCACGTGGAGCTCGGATCC | 56 | Short | 0.005 | No Inhibition |
| aptscl 8 | 12 | CGTACGGTCGACGCTAGCCGGCGAAATT GCTACCACGTGGAGCTCGGATCC | 51 | Short | 0.005 | No Inhibition |
| aptsci 12 | 13 | CGTACGGTCGACGCTAGCCGTTGACTCG TTGCTACACGTGGAGCTCGGATCC | 52 | Short | 0.005 | No Inhibition |
| aptscl 16 | 14 | CGTACGGTCGACGCTAGCTGGTTTCATC GATCGTATCCACGTGGAGCTCGGATCC | 55 | Short | 0.61 | No Inhibition |
| aptscl 22 | 15 | CGTACGGTCGACGCTAGCTGGGTTTATC GACTAGTTCCACGTGGAGCTCGGATCC | 55 | Short | 0.76 | No Inhibition |
| aptscl 29 | 16 | CGTACGGTCGACGCTAGCCGGAGACCTG AGGTTCACGTGGAGCTCGGATCC | 51 | Short | 0.28 | No Inhibition |
| aptscl 32 | 17 | CGTACGGTCGACGCTAGCTGTGTTAATC GCCGTACCTCCACGTGGAGCTCGGATCC | 56 | Short | 0.18 | No Inhibition |
| Antib ody | -- | -- | 145. 9 kDa | | 3.55 | 100 |

### Example 5. Truncation and characterization of aptscl3

Aptscl3 which showed high affinity and inhibition potency to sclerostin was truncated (Table 2). Binding affinity and in vitro inhibition potency were performed using the same protocols in previous studies. Aptscl 3-1, -2, -3, -4 and -5 remained high binding affinity to sclerostin, with the Kd value at 0.86, 0.52, 0.2 and 0.22 nM, respectively. While aptscl 3-6 failed to fit the binding curve at this concentration range, indicating a low binding ability to sclerostin (Figure 6a). Furthermore, aptscl3-5 reserved high inhibition potency to sclerostin's antagonistic effect on Wnt signaling (EC50=28.4 µg/ml) (Figure 6b).

**Table 2 Sequence truncation of aptamer candidate aptscl3**

| **Nam e** | SEQ ID NO | Sequence | **Nucleotide** | **Len gth (nt)** | **Affinity (nM)** |
|---|---|---|---|---|---|
| **apts cl3** | 10 | CGTACGGTCGACGCTAGCTGTTGTACATCGCCT TACGCACGTGGAGCTCGGATCC | 1-55 | 55 | 0.02 |
| **apts cl3-1** | 19 | GTCGACGCTAGCTGTTGTACATCGCCTTACGCA CGTGGAGCTCGGATCC | 7-55 | 49 | 0.86 |
| **apts cl3-2** | 20 | CGTACGGTCGACGCTAGCTGTTGTACATCGCCT TACGCACGTGGAGCTC | 1-49 | 49 | 0.52 |
| **apts cl3-3** | 21 | GCTAGCTGTTGTACATCGCCTTACGCACGTGGA GCTC | 13-49 | 37 | 0.2 |
| **apts cl3-4** | 22 | GTCGACGCTAGCTGTTGTACATCGCCTTACGCA CGTG | 7-43 | 37 | 0.22 |
| **apts cl3-5** | 23 | GCTAGCTGTTGTACATCGCCTTACGCACGTG | 13-43 | 31 | 0.22 |
| **apts cl3-6** | 24 | TGTTGTACATCGCCTTACGCACGTG | 19-43 | 25 | - |

### Example 6. Serum stability assessment of chemically modified aptamer candidates

The inventors had selected DNA aptamers against sclerostin and finally developed two truncated aptamers, termed aptscl 56 and aptscl 3-5, which specifically and tightly bound to sclerostin with a dissociation constant in the low nanomolar range. The bulky 2'-O-methyl (2'-OMe) modifications of nucleic acid aptamer have been previously used as a post-selection modification due to their enhanced nuclease resistance and elevated duplex melting temperature as seen in the clinical examples (Fine, Martin et al. 2005; Gupta, Hirota et al. 2014). The 3'-end capping with inverted dT has also been a common strategy among aptamers for diseases therapy in on-going or completed clinical trials (Padilla, Sousa et al. 1999; Ruckman, Green et al. 1998). Taken together, this example is to evaluate whether the serum stability of aptscl 56 and aptscl 3-5 could be improved by 2'-OMe and 3'-terminal Inverted dT (3'-idT) modifications.

### Experimental design:

The modified nucleotide was introduced during synthesis. The serum metabolic stability of the modified and unmodified aptamers was evaluated in freshly prepared mouse serum. All the aptamer samples were incubated with 10% and 100% mouse serum at 37 °C for 0, 2, 4, 8, 12, 24, 36, 48 and 72 hours, respectively. At the assigned time, the aptamer samples were flash-frozen in a dry ice bath and then stored at -80 °C until all the samples were harvested for evaluation. The stability of all the aptamer samples was expressed as the band density of intact aptamer remaining after the incubation, which could be determined by agarose gel electrophoresis.

### DNA synthesis protocol

Modified and unmodified DNA sequences were synthesized on a 1 µmole scale on a K&A H8 standard DNA/RNA Synthesizer using commercially available 5'-O-DMT-2'-deoxynucleoside (ABz, CAc, GiBu and T) phosphoramidite monomers, 5'-O-DMT-2'-O-methyl nucleoside (ABz, CAc, GiBu and T) phosphoramidite monomers and/or 5'-O-DMT-2'-F-nucleoside (ABz, CAc, GiBu and T) phosphoramidite monomers (Beaucage and Caruthers 2001). The modified sequence of aptscl56 was CGGGG TGTGG GTTCG TCGTT AGCTT GATTT GGCAG CTGCCC-idT and the nucleotide underlined was 2'-OMe modified. The modified sequence of aptscl 3-5 was GCTAG CTGTT GTACA TCGCC TTACG CACGT G-idT and the nucleotide underlined was 2'-OMe modified.

### Evaluation protocols:

The band density of all the aptamer samples was assayed by a molecular imager (Bio-Rad) (Klussmann, Nolte et al. 1996, Siller-Matula, Merhi et al. 2012). The binding affinity and in vitro inhibition potency were performed using the same protocols in previous studies.

### Results:

For aptscl 56, the unmodified aptamer was fully degraded after 48 h in 10% serum and remained only for 8 h in 100% serum. The 2'-OMe and 3' idT modified aptscl 56 was remained for 72h in 10% mouse serum and was degraded after 12h. At 72 h, a small amount of modified aptamer was still remained (Figure 7).

For aptscl 3-5, the unmodified aptamer was degraded 24 h after incubation in 10% mouse serum. The 2'-OMe and 3' idT modified aptscl 3-5 could last 48 h in 10% mouse serum. In 100% serum, the unmodified aptscl 3-5 was rapidly and fully degraded after 8 h, while the modified aptscl 3-5 could remain the integrity after 72 h (Figure 8).

Chemically modified aptscl 56 and aptscl 3-5 showed high binding affinity to sclerostin, with Kd value as 6.55 and 0.54 nM, respectively. Furthermore, chemically modified aptscl 56 and aptscl 3-5 could efficiently inhibit sclerostin's antagonistic effect on Wnt signaling in cells, with the inhibition potency as 14 and 11 µg/ml , respectively (Figure 9).

### Conclusion:

The modification with 2'-OMe and 3' idT could further facilitate developing aptscl 56 and aptscl 3-5 toward therapeutic nuclease-resistant aptamers.

### Example 7. Preparation of fatty acid modified aptamer candidates and in vivo half-life thereof

The nucleic acid aptamer aptscl56, which specifically targets sclerostin, was identified in the above examples. Its specific sequence is:
5'-CGGGGTGTGGGTTCGTCGTTAGCTTGATTTGGCAGCTGCC-3' (SEQ ID No: 1).

The obtained sequence was modified to obtain the sequence APC001 (the 2' position of the sugar ring of the 4 nucleotides at each of the 5' and 3' ends was modified with methoxy; the 3' end of the nucleic acid chain was modified with idT):

C(OMe)G(OMe)G(OMe)G(OMe)GTGTGGGTTCGTCGTTAGCTTGATT
TGGCAGCU(OMe)G(OMe)C(OMe)C(OMe)-idT.

The nucleotide sequence is:
CGGGGTGTGGGTTCGTCGTTAGCTTGATTTGGCAGCCUGCC (SEQ ID NO:25)

In this example, APC001 was conjugated to and modified with a fatty acid (FA) at the 5' end to obtain:

FA-C(OMe)G(OMe)G(OMe)G(OMe)GTGTGGGTTCGTCGTTAGCTTG
ATTTGGCAGCU(OMe)G(OMe)C(OMe)C(OMe)-idT.

The effect of conjugating palmitic acid (PA) and octadecanedioic acid (OA) separately on the in vivo half-life of the aptamer was tested.

### Conjugation method

In the present invention, chemical modification technology was used to perform fatty acid coupling on the 5' end of the adapter nucleic acid chain. Due to the structural similarity of the nucleic acid chains themselves, it is not easy to couple directly on the nucleic acid bases. It is necessary to extend an active amino group at the end of the nucleic acid chain. In the present invention, different aminophosphoramidite monomers were used to construct linking arms to perform amino modification on the end of a nucleic acid chain. These phosphoramidite monomers can be directly incoporated into the nucleic acid solid-phase synthesis cycle to perform coupling reactions to obtain target compounds, specifically as follows :
1) 5'-amino-modifier 5
   2-[2-(4-Monomethoxytrityl)aminoethoxy]ethyl-(2-cyanoethyl)-N,N-diisopropyl)-phosphoramidite 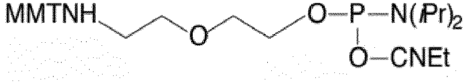 The following structure S1 can be obtained for coupling with the carboxyl group of a fatty acid: 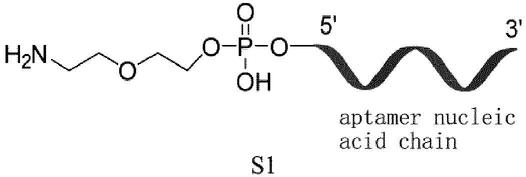
2) 5'-amino-modifier C6
   6-(4-Monomethoxytritylamino)hexyl-(2-cyanoethyl)-(N,N-diisopropyl)-phosphoramidite 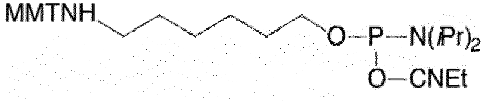 The following structure S2 can be obtained for coupling with the carboxyl group of a fatty acid: 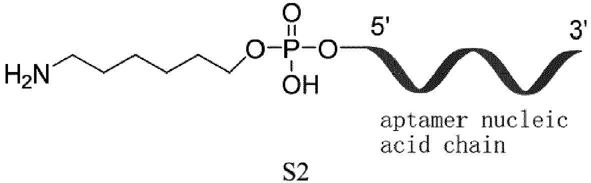
3) 5'-amino-modifier C12
   12-(4-Monomethoxytritylamino)dodecyl-1- [(2-cyanoethyl)-(N,N-diisopropyl)] -phosphoramidite 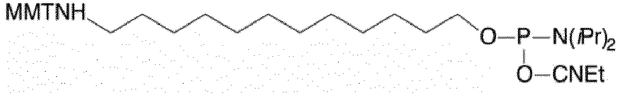 The following structure S3 can be obtained for coupling with the carboxyl group of a fatty acid: 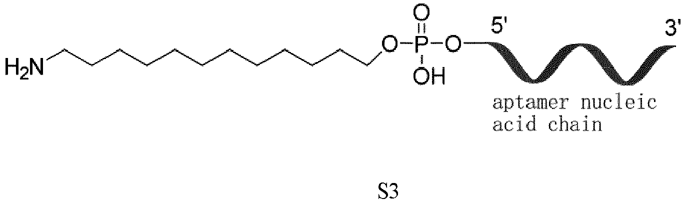
4) 5'-amino-modifier TEG CE-phosphoramidite
   10-(O-trifluoroacetylamido-N-ethyl)-triethylene
   glycol-1-[(2-cyanoethyl)-(N,N-diisopropyl)]-phosphoramidite 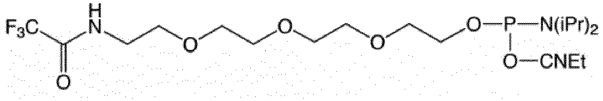 The following structure S4 can be obtained for coupling with the carboxyl group of a fatty acid: 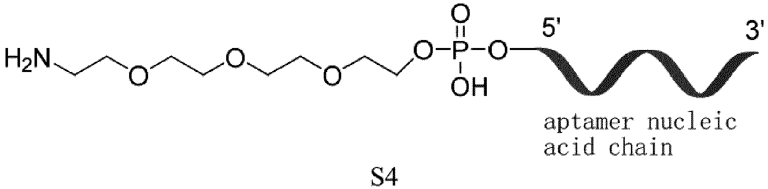
5) 5'-amino-modifier C3-TFA
   3-(Trifluoroacetylamino)propyl-(2-cyanoethyl)-(N,N-diisopropyl)-phosphoramidite 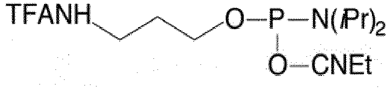 The following structure S5 can be obtained for coupling with the carboxyl group of a fatty acid:

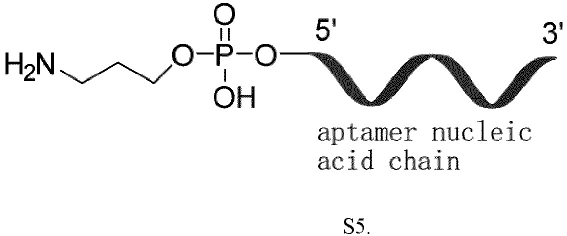

Specifically, the S2 structure of APC001 was used to conduct coupling reactions with palmitic acid and octadecanedioic acid, respectively, to obtain APC001PA and APC001OA.

### In vivo half-life analysis

Pharmacokinetic studies of APC001, APC001PA, and APC001OA were performed in 6-month-old female naive Sprague-Dawley rats fed ad libitum with standard laboratory diet and maintained under controlled conditions (12 h photoperiod, 20°C). Rats were treated with APC001, APC001PA and APC001OA via a single subcutaneous injection. Blood samples were collected from rats in each group at different time points, and plasma was separated. Sangon UNIQ-10 column universal DNA extraction kit was used to extract samples, and the concentrations of APC001, APC001PA and APC001OA in plasma at different time points were measured by HPLC, and then the pharmacokinetic software DS was used to calculate their in vivo half-lives.

The in vivo half-lives of APC001, APC001PA and APC001OA are shown in the table below:

| Name | T_{1/2}(h) |
|---|---|
| APC001 | 1.5 |
| APC001PA | 69.3 |
| APC001OA | 185.1 |

Conjugating aptamers with fatty acids can significantly extend their half-life in vivo.

### Example 8. Preparation of aptamer candidates modified with fatty acids and coumarin derivatives, and in vivo half-life thereof

### 1. Virtual screening of coumarin derivatives:

Warfarin (WA) has a strong affinity with serum albumin (HSA), but due to the anticoagulant effect of WA, it is not suitable as a small molecule coupling reagent. Through a virtual high-throughput screening method, the present invention identified 4-hydroxycoumarin with high affinity for HSA and no anticoagulant function (Table 3). Virtual high-throughput screening was performed using Schrodinger_Suites_2021-2 software, which is a software that can simulate small molecule and macromolecule systems and was used to perform docking simulations of selected compounds with full-length HSA and FABP respectively.

**Table 3 The top ten predicted warfarin derivatives with the best human**

| Structure | Binding Energy | RSA_Rank | Structure | Binding Energy | RSA_Rank |
|---|---|---|---|---|---|
| 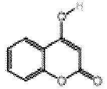 4-Hydroxycoumarin (HC) | -7.28 | 1 | 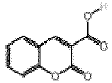 Coumarin-3-carboxylic acid | -6.78 | 6 |
| 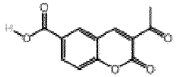 3-Acetyl-6-carboxycoumarin | -7.14 | 2 | 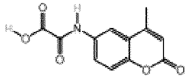 N-(4-Methyl-7-coumarinyl) oxalic acid amide | -6.58 | 7 |
| 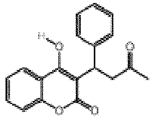 Warfarin | -6.97 | 3 | 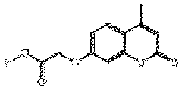 7-(Carboxymethoxy)-4-methylcoumarin | -6.37 | 8 |
| 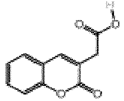 (2-Oxo-2H-chromen-3-yl) acetic acid | -6.86 | 4 | 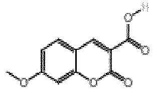 7-Methoxycoumarin-3-carboxylic acid | -6.35 | 9 |
| 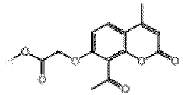 [(8-acetyl-4-methyl-2-oxo-2H-chromen-7-yl)oxy]acetic acid | -6.81 | 5 | 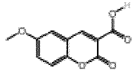 6-Methoxy-2-oxo-2H-chromene-3-carboxylic acid | -6.26 | 10 |

| | | | | | |
|---|---|---|---|---|---|
| serum albumin (HSA) binding affinity | | | | | |

### 2. Affinity verification of 4-hydroxycoumarin and HSA and anticoagulant function verification:

The affinity of warfarin and 4-hydroxycoumarin to HSA was determined by surface plasmon resonance (SPR) technology. 4-Hydroxycoumarin was found to have a slightly stronger affinity for HSA than warfarin. And according to the thrombin time (TT) measurement test, it was found that 4-hydroxycoumarin has no anticoagulant function. The results are shown in Figure 10.

### 3. Virtual screening of fatty acid derivatives:

Octadecanedioic acid (OA) has a strong affinity with serum albumin (HSA), but because OA can also bind to fatty acid binding protein (FABP), causing off-target effects, it is not suitable as a small molecule coupling reagent. Through a virtual high-throughput screening method, the present invention obtained dodecanedioic acid (DA) with high affinity for HSA and low affinity for FABP (Table 4). Virtual high-throughput screening was performed using Schrodinger_Suites_2021-2 software, which is software that can simulate small molecule and macromolecule systems and was used to perform docking simulations of selected compounds with full-length HSA and FABP respectively.

**TABLE 4 Truncated fatty acid and their binding ability to FABP and HSA**

| Entry | Structure | Binding Energy (to HSA) | RSA_Rank (to HSA) | Binding Energy (to FABP) | RSA_Rank (to FABP) |
|---|---|---|---|---|---|
| **18C** | 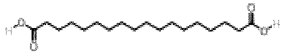 | -9.49 | 1 | -3.85 | 3 |
| **12C (DA)** | 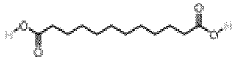 | -7.58 | 2 | -0.23 | 9 |
| **14C** | 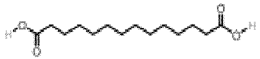 | -7.58 | 3 | -1.75 | 6 |
| **17C** | 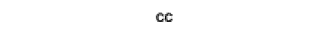 | -7.57 | 4 | -4.08 | 2 |
| **13C** | 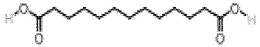 | -7.53 | 5 | -1.61 | 7 |
| **16C** | 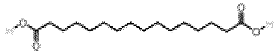 | -7.35 | 6 | -1.34 | a |
| **15C** | 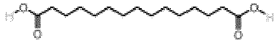 | -7.27 | 7 | -2.73 | 5 |
| **11C** | 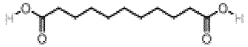 | -7.10 | 8 | -3.7 | 4 |
| **10C** | 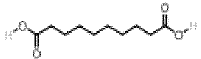 | -5.40 | 9 | -4.16 | 1 |

### 4. Affinity verification of dodecanedioic acid and HSA:

The affinity of dodecanedioic acid and octadecanedioic acid to HSA and FABP, respectively, was determined by surface plasmon resonance (SPR) technology. Dodecanedioic acid was found to have a slightly stronger affinity for HSA than octadecanedioic acid, and a weaker affinity for FABP than octadecanedioic acid. The results are shown in Figure 11.

### 5. Coupling method

In the present invention, chemical modification technology was used to couple fatty acids and coumarin derivatives to the 5' end of the adapter nucleic acid chain. Due to the structural similarity of the nucleic acid chains themselves, it is not easy to couple directly on the nucleic acid bases, and an active group needs to be extended at the end of the nucleic acid chain. In the present invention, the following 5'-amino-modifier C7-TFA monomer was used to build a connecting arm to modify the end of the nucleic acid chain,
5'- amino - modifier C7-TFA

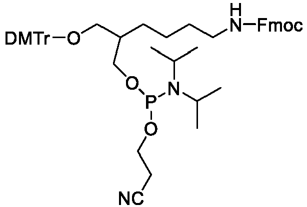

The following structure S6 was obtained:

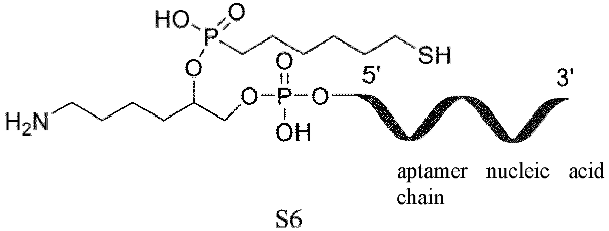

Then 4-hydroxycoumarin was connected to the sulfhydryl group of S6, and dodecanedioic acid was connected to the amino group of S6.

### 6. In vivo half-life detection of APC001 conjugated with 4-hydroxycoumarin and dodecanedioic acid

APC001 conjugated with 4-hydroxycoumarin and dodecanedioic acid was named APC001OC, and its structure is as follows: 
OC-C(OMe)G(OMe)G(OMe)G(OMe)GTGTGGGTTCGTCGTTAGCTTG
ATTTGGCAGCU(OMe)G(OMe)C(OMe)C(OMe)-idT
 where OC represents conjugated 4-hydroxycoumarin and dodecanedioic acid. The pharmacokinetic study method is similar to the previous example.

The in vivo half-life results for the 4-hydroxycoumarin and dodecanedioic acid double-modified sclerostin nucleic acid aptamer and its derivatives disclosed in the present invention as compared with the APC001 without the 4-hydroxycoumarin and dodecanedioic acid double-modification are shown as follows:

| Name | T_{1/2} (h) |
|---|---|
| APC001 | 1.5 |
| APC001OA | 185.1 |
| APC001HC | 162.93 |
| APC001DA | 98.81 |
| APC001OC | 289.94 |

wherein, OA represents octadecanedioic acid modification, HC represents 4-hydroxycoumarin modification, DA represents dodecanedioic acid modification, and OC represents 4-hydroxycoumarin and dodecanedioic acid modification.

### 7. In vivo half-life detection of aptamers against DKK1 conjugated with 4-hydroxycoumarin and dodecanedioic acid

The sequence of the aptamer against DKK1 (APTDKK1) is as follows:
CGTACGGTCGACGCTAGCTGGTGGTTGGGGTGGGTGGT(SEQ ID NO:26)

APTDKK1 conjugated with 4-hydroxycoumarin and dodecanedioic acid is named APTDKK1OC, and its structure is as follows:
OC-CGTACGGTCGACGCTAGCTGGTGGTTGGGGTGGGTGGT
where OC represents conjugated 4-hydroxycoumarin and dodecanedioic acid. The pharmacokinetic study method is similar to the previous example.

The in vivo half-life results for the 4-hydroxycoumarin and dodecanedioic acid double-modified DKK1 nucleic acid aptamer and its derivatives disclosed in the present invention as compared with the APTDKK1 without the 4-hydroxycoumarin and dodecanedioic acid double-modification are shown as follows:

| Name | T_{1/2} (h) |
|---|---|
| APTDKK1 | 1.2 |
| APTDKK1OA | 178.5 |
| APTDKK1HC | 134.2 |
| APTDKK1DA | 72.3 |
| APTDKK1OC | 234.5 |

wherein, OA represents octadecanedioic acid modification, HC represents 4-hydroxycoumarin modification, DA represents dodecanedioic acid modification, and OC represents 4-hydroxycoumarin and dodecanedioic acid modification.

## Claims

**1.** A nucleic acid molecule conjugate with extended in vivo half-life, wherein the nucleic acid molecule is conjugated to a fatty acid and/or a coumarin derivative.

**2.** The nucleic acid molecule conjugate of claim 1, wherein the fatty acid is selected from dodecanedioic acid, palmitic acid (PA), tetradecanedioic acid, hexadecanedioic acid, stearic acid (SA), octadecanedioic acid, lauric acid, acid, eicosapentaenoic acid (EPA), docosahexaenoic acid (DHA), and arachidonic acid (ARA), preferably, the fatty acid is dodecanedioic acid.

**3.** The nucleic acid molecule conjugate of claim 1 or 2, wherein the coumarin derivative is selected from 4-hydroxycoumarin, 3-acetyl-6-carboxycoumarin, warfarin, (2-oxo-2H-chromen-3-yl)acetic acid, [(8-acetyl-4-methyl- 2-oxo-2H-chromen-7-yl)oxy] acetic acid, coumarin-3-carboxylic acid, N-(4-methyl-7-coumarin) oxalamide, 7-(carboxymethyl )-4-methylcoumarin, 7-methoxycoumarin-3-carboxylic acid, and 6-methoxy-2-oxo-2H-chromene-3-carboxylic acid, preferably, the coumarin derivative is 4-hydroxycoumarin.

**3.** The nucleic acid molecule conjugate of any one of claims 1-3, wherein the fatty acid, such as dodecanedioic acid, is conjugated to the 5' end of the nucleic acid molecule; or the coumarin derivative, such as 4-hydroxycoumarin, is conjugated to the 5' end of the nucleic acid molecule; or the fatty acid, such as dodecanedioic acid, and the coumarin derivative, such as 4-hydroxycoumarin, are conjugated to the 5' end of the nucleic acid molecule.

**5.** The nucleic acid molecule conjugate of any one of claims 1-4, wherein the fatty acid, such as dodecanedioic acid, is conjugated to the nucleic acid molecule through a linker; or the coumarin derivative, such as 4-hydroxycoumarin, is conjugated to the nucleic acid molecule via a linker; or the fatty acid, such as dodecanedioic acid, and the coumarin derivative, such as 4-hydroxycoumarin, are conjugated to the nucleic acid molecule through a linker.

**6.** The nucleic acid molecule conjugate of any one of claims 1-5, wherein the nucleic acid molecule is a DNA molecule, an RNA molecule, or a DNAJRNA hybrid molecule.

**7.** The nucleic acid molecule conjugate of any one of claims 1-6, wherein the nucleic acid molecule is double-stranded; or the nucleic acid molecule is single-stranded; or the nucleic acid molecule contains a single-stranded portion and a double-stranded portion.

**8.** The nucleic acid molecule conjugate of any one of claims 1-7, wherein the nucleic acid molecule is about 1 to about 250 bp/nt, about 1 to about 200 bp/nt, about 1 to about 150 bp/nt, about 1 to about 100 bp/nt, about 1 to about 50 bp /nt, about 1 to about 30bp/nt, about 1 to about 20bp/nt, about 1 to about 15bp/nt, about 1 to about 10bp/nt in length.

**9.** The nucleic acid molecule conjugate of any one of claims 1-8, wherein the nucleic acid molecule is an aptamer, siRNA, antisense RNA, or shRNA, preferably, the nucleic acid molecule is an aptamer.

**10.** The nucleic acid molecule conjugate of any one of claims 1-9, wherein the nucleic acid molecule comprises one or more modifications that confers enhanced nuclease resistance to the nucleic acid molecule.

**11.** The nucleic acid molecule conjugate of claim 10, wherein the modification includes a 3' inverted deoxythymidine (3' idT) modification.

**12.** The nucleic acid molecule conjugate of claim 10, wherein the modification includes substituting one or more naturally occurring nucleotides with modified nucleotides selected from the group consisting of 2'-fluoro, 2'-methoxyethyl, 2'-methoxy or 2' allyloxy modified nucleotides, preferably 2'-methoxy modified nucleotides.

**13.** The nucleic acid molecule conjugate of claim 10, wherein the modification includes an internucleotide modification, such as an internucleotide phosphorothioate bond modification.

**14.** The nucleic acid molecule conjugate of claim 10, wherein the aptamer comprises a 2'-methoxy (2'-OMe) modification, and/or a 3' inverse deoxythymidine (3' idT) modification.

**15.** The nucleic acid molecule conjugate of any one of claims 1-14, wherein the in vivo half-life of the nucleic acid molecule conjugate is at least 2 times, at least 5 times, at least 10 times, at least 25 times, at least 50 times, at least 100 times, at least 200 times or more as compared to a corresponding nucleic acid molecule that does not comprise the conjugated fatty acid and/or coumarin derivative

**16.** A aptamer conjugate against sclerostin, which comprises an aptamer against sclerostin conjugated to a fatty acid and/or a coumarin derivative,
wherein the aptamer comprises
i) a nucleotide sequence having at least about 90% identity, at least about 91% identity, at least about 90% identity, about 92% identity, at least about 93% identity, at least about 94% identity, or at least about 95% identity to any one of SEQ ID NOs:1-17, or
ii) at least 30, at least 35, at least 40, at least 45, at least 50, or more consecutive nucleotides within any one of SEQ ID NOs: 1-17,
wherein the aptamer specifically binds to sclerostin.

**17.** The aptamer conjugate of claim 16, wherein the aptamer comprises a nucleotide sequence of any one of SEQ ID NOs: 1-17 and 19-25.

**18.** The aptamer conjugate of claim 16 or 17, wherein the aptamer is conjugated to a fatty acid and a coumarin derivative.

**19.** The aptamer conjugate of any one of claims 16-18, wherein the fatty acid is selected from dodecanedioic acid, palmitic acid (PA), tetradecanedioic acid, hexadecanedioic acid, stearic acid (SA), octadecanedioic acid, lauric acid, acid, eicosapentaenoic acid (EPA), docosahexaenoic acid (DHA), and arachidonic acid (ARA), preferably, the fatty acid is dodecanedioic acid.

**20.** The aptamer conjugate of any one of claims 16-19, wherein the coumarin derivative is selected from 4-hydroxycoumarin, 3-acetyl-6-carboxycoumarin, warfarin, (2-oxo-2H-chromen-3-yl)acetic acid, [(8-acetyl-4-methyl- 2-oxo-2H-chromen-7-yl)oxy] acetic acid, coumarin-3-carboxylic acid, N-(4-methyl-7-coumarin) oxalamide, 7-(carboxymethyl )-4-methylcoumarin, 7-methoxycoumarin-3-carboxylic acid, and 6-methoxy-2-oxo-2H-chromene-3-carboxylic acid, preferably, the coumarin derivative is 4-hydroxycoumarin.

**21.** The aptamer conjugate of any one of claims 16-20, wherein the fatty acid, such as dodecanedioic acid, is conjugated to the 5' end of the aptamer; or the coumarin derivative, such as 4-hydroxycoumarin, is conjugated to the 5' end of the aptamer; or the fatty acid, such as dodecanedioic acid, and the coumarin derivative, such as 4-hydroxycoumarin, are conjugated to the 5' end of the aptamer.

**22.** The aptamer conjugate of any one of claims 16-21, wherein the fatty acid, such as dodecanedioic acid, is conjugated to the aptamer through a linker; or the coumarin derivative, such as 4-hydroxycoumarin, is conjugated to the aptamer via a linker; or the fatty acid, such as dodecanedioic acid, and the coumarin derivative, such as 4-hydroxycoumarin, are conjugated to the aptamer through a linker.

**23.** The aptamer conjugate of any one of claims 16-22, wherein the aptamer has a Kd to sclerostin of less than 100 nM, preferably less than 50 nM, preferably less than 40 nM, preferably less than 30 nM, preferably less than 20 nM, preferably less than 10 nM or less.

**24.** The aptamer conjugate of any one of claims 16-23, wherein the aptamer can inhibit the biological activity of sclerostin.

**25.** The aptamer conjugate of any one of claims 16-24, wherein the aptamer can block the antagonistic effect of sclerostin in a cell-based Wnt signaling assay.

**26.** The aptamer conjugate of any one of claims 16-25, wherein the aptamer inhibits the biological activity of sclerostin, such as inhibits the antagonistic effect of sclerostin on the Wnt signaling pathway with a EC50 value of less than 100 µg/ml, preferably less than 50 µg/ml, preferably less than 40 µg/ml, preferably less than 30 µg/ml, preferably less than 20 µg/ml, preferably less than 10 µg/ml or less.

**27.** The aptamer conjugate of any one of claims 16-26, wherein the aptamer comprises one or more modifications that confers enhanced nuclease resistance to the nucleic acid molecule.

**28.** The aptamer conjugate of claim 27, wherein the modification includes a 3' inverted deoxythymidine (3' idT) modification.

**29.** The aptamer conjugate of claim 27, wherein the modification includes substituting one or more naturally occurring nucleotides with modified nucleotides selected from the group consisting of 2'-fluoro, 2'-methoxyethyl, 2'-methoxy or 2' allyloxy modified nucleotides, preferably 2'-methoxy modified nucleotides.

**30.** The aptamer conjugate of claim 27, wherein the modification includes an internucleotide modification, such as an internucleotide phosphorothioate bond modification.

**31.** The aptamer conjugate of claim 27, wherein the aptamer comprises a 2'-methoxy (2'-OMe) modification, and/or a 3' inverse deoxythymidine (3' idT) modification.

**32.** A method for treating sclerostin-related diseases, the method comprises administering a therapeutically effective amount of the aptamer conjugate against sclerostin of any one of claims 16-31 to a subject in need thereof, for example, the subject is a human.

**33.** The method of claim 32, wherein the sclerostin-related disease is selected from osteoporosis, osteopenia, osteomalacia, osteogenesis imperfecta (OI), ischemic osteonecrosis, rheumatoid arthritis, fracture, osteoarthritis and myeloma, hypophosphatemic rickets and triple negative breast cancer.

**34.** A pharmaceutical composition comprising at least one aptamer conjugate against sclerostin of any one of claims 16-31, and a pharmaceutically acceptable carrier or excipient.

**35.** Use of the aptamer conjugate against sclerostin of any one of claims 16-31 or the pharmaceutical composition of claim 34 in the preparation of a medicine for treating sclerostin-related diseases.

**36.** The use of claim 35, wherein the sclerostin-related disease is selected from osteoporosis, osteopenia, osteomalacia, osteogenesis imperfecta (OI), ischemic osteonecrosis, rheumatoid arthritis, fracture, osteoarthritis and myeloma, hypophosphatemic rickets and triple negative breast cancer.
